# EUROPEAN PATENT SPECIFICATION

(11) **EP 4 372 382 B1**
(45) Date of publication and mention of the grant of the patent: **28.10.2026**
(21) Application number: 23162383.6
(22) Date of filing: 16.03.2023
(51) Int. Cl.: G01N 33/68

(54) **METHODS FOR COLORECTAL CANCER DIAGNOSIS AND PROGNOSIS**
VERFAHREN ZUR DIAGNOSE UND PROGNOSE VON KOLOREKTALKARZINOM
PROCÉDÉS DE DIAGNOSTIC ET DE PRONOSTIC DU CANCER COLORECTAL

(30) Priority: 15.11.2022 TW 111143627
(43) Date of publication of application: 22.05.2024
(73) Proprietor: S&T BIOMED CO., LTD., 302041, Zhubei City, Hsinchu County (TW)
(72) Inventor: YU, Jau-Song, 333 Taoyuan City (TW); DASH, Srinivas, 333 Taoyuan City (TW); WU, Chia-Chun, 333 Taoyuan City (TW); CHIANG, Sheng-Fu, 333 Taoyuan City (TW); LU, Yu-Ting, 333 Taoyuan City (TW)
(74) Representative: Lang, Christian

(56) References cited:
- WO-A1-2014/097584
- US-A1- 2012 087 862
- US-A1- 2016 041 153
- LARISSA BELOV ET AL: "Cell Surface Markers in Colorectal Cancer Prognosis", INTERNATIONAL JOURNAL OF MOLECULAR SCIENCES, vol. 12, no. 1, 28 December 2010 (2010-12-28), pages 78 - 113, XP055275867, DOI: 10.3390/ijms12010078
- DONG-SIC CHOI ET AL: "Quantitative proteomics of extracellular vesicles derived from human primary and metastatic colorectal cancer cells. v", JOURNAL OF EXTRACELLULAR VESICLES, 11 September 2012 (2012-09-11), pages 1 - 15, XP055305776, DOI: 10.3402/jev.v1i0.18704
- NICHOLAS F S WATSON ET AL: "Expression of the membrane complement regulatory protein CD59 (protectin) is associated with reduced survival in colorectal cancer patients", CANCER IMMUNOLOGY, IMMUNOTHERAPY, SPRINGER, BERLIN, DE, vol. 55, no. 8, 3 September 2005 (2005-09-03), pages 973 - 980, XP019333271, ISSN: 1432-0851, DOI: 10.1007/S00262-005-0055-0
- GLASS SARAH E ET AL: "Recent Advances in the Study of Extracellular Vesicles in Colorectal Cancer", GASTROENTEROLOGY, ELSEVIER INC, US, vol. 163, no. 5, 18 June 2022 (2022-06-18), pages 1188 - 1197, XP087204209, ISSN: 0016-5085, [retrieved on 20220618], DOI: 10.1053/J.GASTRO.2022.06.039
- CHEN HANG ET AL: "Quantitative analysis of wild-type and V600E mutant BRAF proteins in colorectal carcinoma using immunoenrichment and targeted mass spectrometry", ANALYTICA CHIMICA ACTA, ELSEVIER, AMSTERDAM, NL, vol. 933, 2 June 2016 (2016-06-02), pages 144 - 155, XP029672879, ISSN: 0003-2670, DOI: 10.1016/J.ACA.2016.05.037
- DASH SRINIVAS ET AL: "Extracellular Vesicle Membrane Protein Profiling and Targeted Mass Spectrometry Unveil CD59 and Tetraspanin 9 as Novel Plasma Biomarkers for Detection of Colorectal Cancer", CANCERS, vol. 15, no. 1, 28 December 2022 (2022-12-28), pages 1 - 27, XP093073776, DOI: 10.3390/cancers15010177

## Description

### BACKGROUND OF THE INVENTION

### 1. FIELD OF THE INVENTION

The present invention relates to a method for determining whether an human has or is at risk of developing colorectal cancer (CRC) with an extracellular vesicle (EV) derived from an ex vivo biological sample isolated from the human, comprising use of two or three target proteins for detecting colorectal cancer (CRC); more particularly, the method includes the step of determining the two target proteins of CD59 and TSPAN9, or the three target proteins of ADAM10, CD59 and TSPAN9 in an extracellular vesicle (EV) to serve as a tool for detecting CRC by using plasma samples isolated from human patients.

### 2. DESCRIPTION OF THE RELATED ART

Colorectal cancer (CRC) is the third most common cancer worldwide and is the second most deadly cancer with an incidence rate of two million new CRC cases and one million deaths, which accounts for around one in every ten cancer cases and deaths. According to the US Centers for Disease Control and Prevention (CDC), those diagnosed with localized CRC have a 5-year survival probability of 91%, as compared to that of 70% for a regional-spread stage and that of 11% for a distant-spread stage. Recently, data from the CDC shows that CRC incidence and mortality have decreased in the last few years, which is attributed to the benefits of screening initiatives; nonetheless, current measures for diagnosing CRC are colonoscopies and biopsies, which are extremely invasive procedures. Alternative diagnostic methods for CRC with low invasiveness have been developing for years, in hoping to replace the high-invasive examination methods of the past. One of the feasible ways is to develop "liquid biopsies," which are body fluids with molecular signals therein, to be used as a sample in diagnostic methods for CRC, so as to solve many of the problems associated with a sample derived from traditional tissue samplings (e.g., invasiveness or tumor heterogeneity). Liquid biopsies are non-invasive and have evolved into a good screening source for stage-specific diagnosis, medication response, and disease severity. Note that human fluids are allowed for use in early detection of diseases.

Larissa Belov et al, in International Journal of Melecular Sciences, Vol. 12, No. 1, 28 December 2010 describes, very generally, quantification of cell surface markers ADAM10 and CD59 from tumor-derived microvesicles for colorectal cancer prognosis.

Given that CRC is of highly genetic heterogeneity, a panel of biomarkers would be required to achieve sufficient sensitivity and specificity for clinical application of screening. Although there are a bunch of independent biomarker candidates for detecting CRC have been proposed in recent decades, very few of them have been carefully evaluated and quantitatively compared in parallel, in an effort to identify which candidates to form the panel of biomarkers that could be subjected to further clinical validation in a large sample cohort. This may lead to no such panel of molecular biomarkers yet been approved by an official health agency to aid in the early detection and/or management of CRC to date. In view of the foregoing, there exists in the related art a need for a novel panel of biomarkers for making a diagnosis and/or prognosis of CRC so that the human in need thereof could receive a suitable therapeutic regimen in time.

### SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

The present invention is defined in claim 1 and is directed to a method for determining whether a human has or is at risk of developing CRC in the EV derived from an ex vivo biological sample isolated from the human. The method comprises the steps of:
(a) determining a concentration of (1) two target proteins of CD59 and TSPAN9 or (2) three target proteins of ADAM10, CD59, and TSPAN9 in the EV derived from the ex vivo biological sample by the steps of:
   (a-1) selecting two or three surrogate peptides corresponding to the two or three target proteins, wherein each of the two or three surrogate peptides is selected from the group consisting of ADAM10 surrogate peptide, CD59 surrogate peptide, and TSPAN9 surrogate peptide, wherein the ADAM10 surrogate peptide comprises the amino acid sequence of SEQ ID NO: 1; the CD59 surrogate peptide comprises the amino acid sequence of SEQ ID NO: 6; and the TSPAN9 surrogate peptide comprises the amino acid sequence of SEQ ID NO: 12;
   (a-2) labeling the two or three surrogate peptides of step (a-1) by isotopes;
   (a-3) digesting the EV derived from the ex vivo biological sample by means of a proteolytic process to produce a digest;
   (a-4) adding a predetermined concentration of the isotope-labeled two or three surrogate peptides of step (a-2) to the digest of step (a-3);
   (a-5) determining the amounts of the two or three target peptides and the isotope-labeled two or three surrogate peptides in the mixture of step (a-4) by mass spectrometry;
   (a-6) dividing the determined amounts of the two or three target peptides by the determined amounts of the isotope-labeled two or three surrogate peptides to produce a ratio; and
   (a-7) determining the concentration of the two or three target proteins in the EV derived from the ex vivo biological sample based on the ratio of step (a-6) and the predetermined concentration of the isotope-labeled two or three surrogate peptides of step (a-4);
(b) calculating a risk score based on the concentrations of the two or three target proteins determined in step (a); and
(c) determining whether the human has or is at risk of developing CRC based on the calculated risk score of step (b), wherein the human does not have or is at low risk of developing CRC if the calculated risk score of step (b) is lower than a predetermined risk score, and the human has or is at high risk of developing CRC if the calculated risk score of step (b) is the same or above the predetermined risk score.

According to some embodiments of the present disclosure, in step (b), the risk score is calculated by use of logistic regression. Preferably, the risk score is calculated by an equation of: $risk score = \frac{{e}^{\left(a+b1X1+b2X2+b3X3\right)}}{1 + {e}^{\left(a+b1X1+b2X2+b3X3\right)}}$ wherein e is a mathematical constant that is the base of the natural logarithm; a is a constant value; X1, X2, and X3 respectively represent the concentrations of ADAM10, CD59, and TSPAN9; and b1, b2, and b3 respectively represent the coefficient of variation of ADAM10, CD59, and TSPAN9.

According to some embodiments of the present disclosure,
(1) in the case when the at least two target proteins of step (a) are CD59 and TSPAN9, the predetermined risk score of step (c) is 0.211, or
(2) in the case when the at least three target proteins of step (a) are ADAM10, CD59, and TSPAN9, and the predetermined risk score of step (c) is 0.238.

According to the embodiments of the present disclosure, the ex vivo biological sample comprises an extracellular vesicle (EV), which may be derived from blood, plasma, serum, saliva, sputum, urine, ascites, cerebrospinal fluid, amniotic fluid, or tissue lysate of the human.

According to the present invention, the method determines whether a human has or is at risk of developing colorectal cancer (CRC).

According to the preferred embodiments of the present disclosure, the level of each target proteins is determined by liquid chromatography-tandem mass spectrometry (LC-MS/MS). Based on the quantified result, a predetermined risk score can be generated and serves as an indicator of CRC. According to embodiments of the present disclosure, when the calculated risk score of step (b) is lower than the predetermined risk score (e.g., < 0.2), then the human does not have CRC or is at low risk of developing CRC; and when the calculated risk score of step (b) is the same or above the predetermined risk score (e.g., ≥ 0.2), then the human has CRC or is at high risk of developing CRC.

Many of the attendant features and advantages of the present disclosure will becomes better understood with reference to the following detailed description considered in connection with the accompanying drawings.

### BRIEF DESCRIPTION OF THE DRAWINGS

The present description will be better understood from the following detailed description read in light of the accompanying drawings, where:

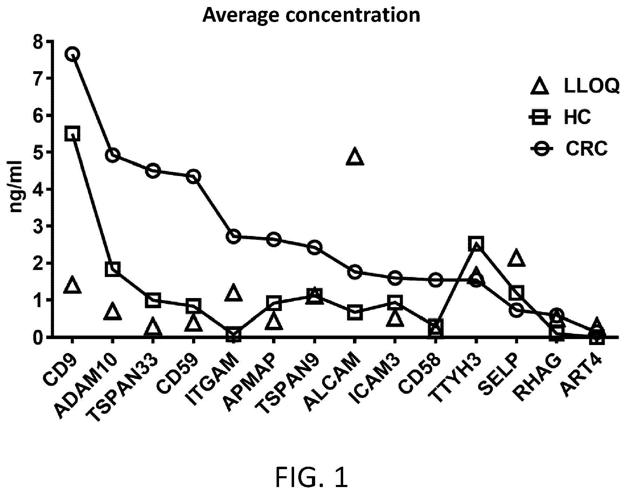

FIG. 1 is the result of comparing the average concentration of 14 targets in the individual plasma EV sample isolated from the healthy control (HC) humans (n = 80) and the CRC patients (n = 73) with the lower limit of quantification (LLOQ) value of each corresponding protein.

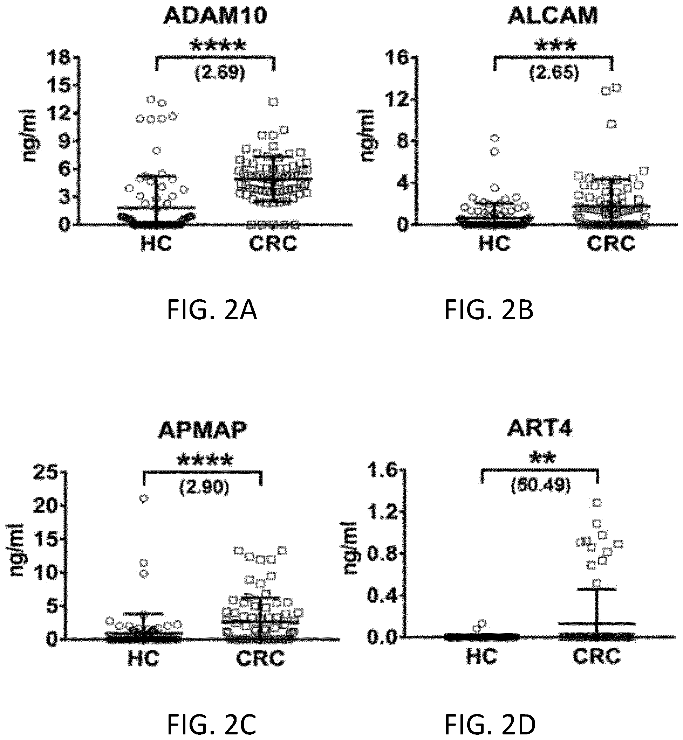

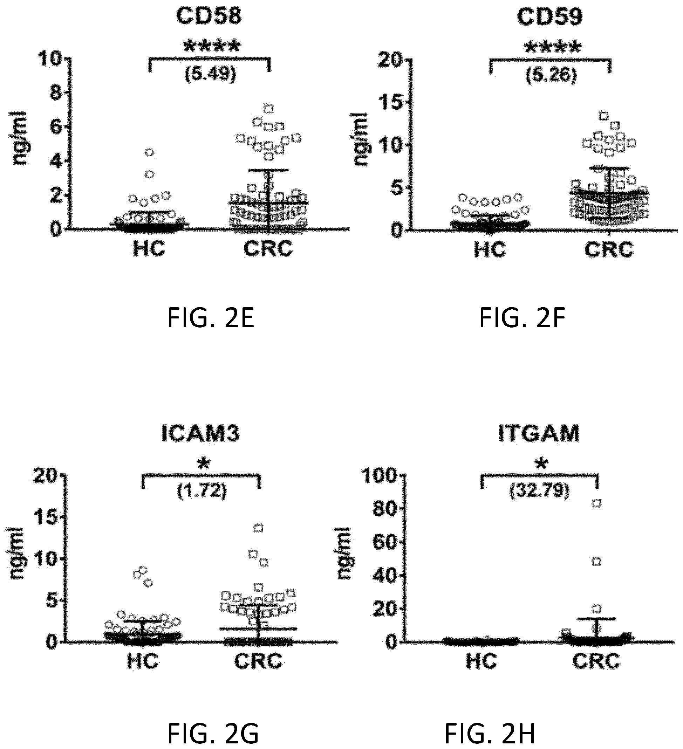

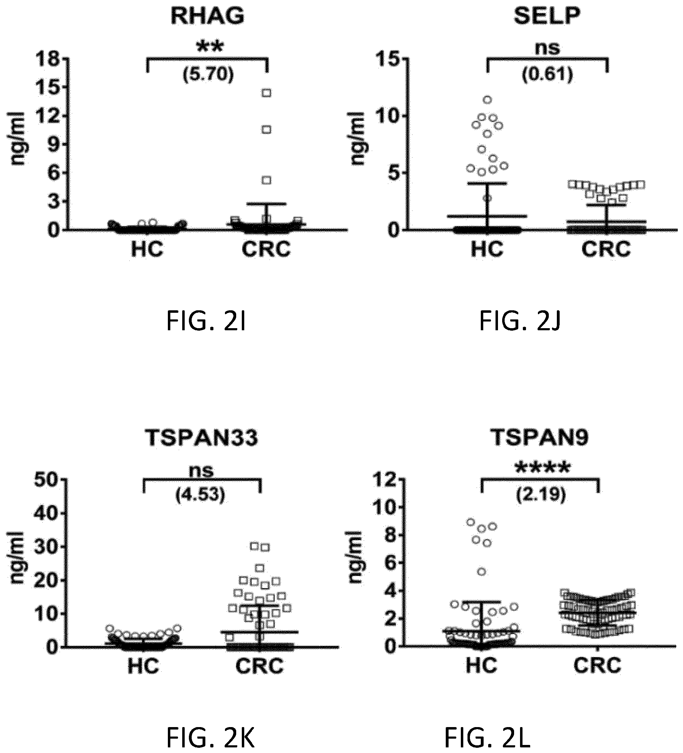

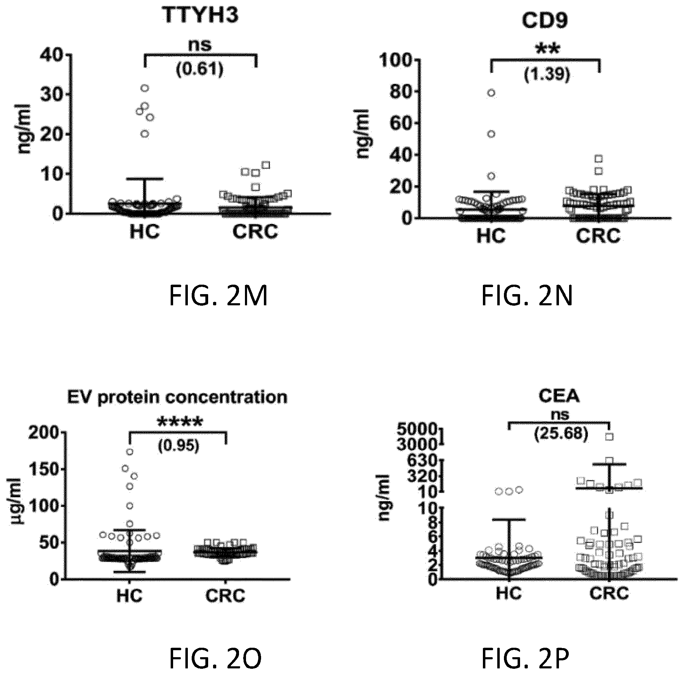

FIGs. 2A-2P illustrate the respective levels of 13 proteins (FIGs. 2A-2M), the level of CD9 (FIG. 2N), and the concentration of the total protein (FIG. 2O) in the individual plasma EVs isolated from the HC humans (n = 80) and the CRC patients (n = 73); and the level of CEA (FIG. 2P) in the individual plasma specimens isolated from the HC humans (n = 80) and the CRC patients (n = 73). The horizontal lines indicate mean ± S.D.; **, p* ≤ 0.05; ***,p* ≤ 0.01; ****, p* ≤ 0.001; *****, p* ≤ 0.0001; ns, not significant.

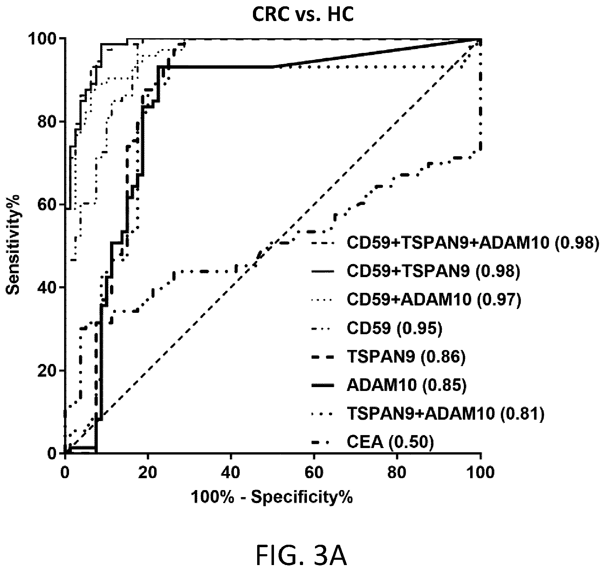

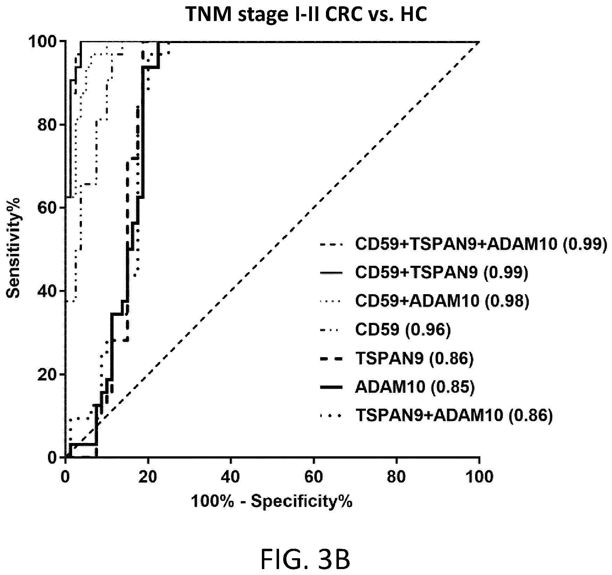

FIGs. 3A-3B depict the results of the receiver operating characteristic (ROC) curve analysis of the indicated panel of the biomarkers, showing the area under the ROC curve (AUC), the sensitivity, and the specificity, in distinguishing the CRC patients (FIG. 3A) or the CRC patients with TNM stage I and II (FIG. 3B) from the HC humans.

### DETAILED DESCRIPTION OF THE INVENTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

### I. Definitions

For convenience, certain terms employed in the specification, examples and appended claims are collected here. Unless otherwise defined herein, scientific and technical terminologies employed in the present disclosure shall have the meanings that are commonly understood and used by one of ordinary skill in the art. Also, unless otherwise required by context, it will be understood that singular terms shall include plural forms of the same and plural terms shall include the singular. Specifically, as used herein and in the claims, the singular forms "a," "an," and "the" include the plural reference unless the context clearly dictates otherwise. Also, as used herein and in the claims, the terms "at least one" and "one or more" have the same meaning and include one, two, three, or more. The practice of the present invention will employ, unless otherwise indicated, conventional techniques of biochemistry, molecular biology, and the like, which are within the skill of the art. Such techniques are explained fully in the literature.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B," when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about". Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

"Percentage (%) amino acid sequence identity" with respect to the polypeptide sequences identified herein is defined as the percentage of polypeptide residues in a candidate sequence that are identical with the amino acid residues in the specific polypeptide sequence, after aligning the sequences and introducing gaps, if necessary, to achieve the maximum percent sequence identity, and not considering any conservative substitutions as part of the sequence identity. Alignment for purposes of determining percentage sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, sequence comparison between two polypeptide sequences was carried out by computer program Blastp (protein-protein BLAST) provided online by Nation Center for Biotechnology Information (NCBI). The percentage amino acid sequence identity of a given polypeptide sequence A to a given polypeptide sequence B (which can alternatively be phrased as a given polypeptide sequence A that has a certain % amino acid sequence identity to a given polypeptide sequence B) is calculated by the formula as follows: $\frac{X}{Y} \times 100 %$ where X is the number of amino acid residues scored as identical matches by the sequence alignment program BLAST in that program's alignment of A and B, and where Y is the total number of amino acid residues in A or B, whichever is shorter.

The term "receiver operating characteristic (ROC) curve" as used herein refers to a plot of the true positive rate against the false positive rate for determining a possible cut-off point of a prognostic or diagnostic test. An ROC consists of graphing (1 - specificity) on the x-axis vs. the sensitivity values on the y-axis. A high sensitivity results in low number of false negative cases. A high specificity refers to low number of false positive cases. The term "cut-off point" refers to a number obtained from an ROC representing a balance between sensitivity and specificity of the prognostic or diagnostic test. A cut-off range can encompass a number of cut-off embodiments, where each represents a different balance between sensitivity and specificity.

The term "area under the ROC curve (AUC)" is used in its art accepted manner and is defined as the area under the ROC curve. An AUC ranging between 0.5-1.0 is a measure for the accuracy of a prognostic or diagnostic test, in which the higher the AUC value, the better the performance of the prognostic or diagnostic test. The AUC value is often presented along with its 95% confidence interval (CI) that refers to a statistical range with a specified probability that a given parameter lies within the range.

Throughout the present disclosure, the term "assessing" refers to a process in which the health status of a human is determined. The health status of the human may indicate a diagnosis, prognosis, or increased risk of a cancer in said human.

The term "risk" herein refers to the potential that a result will lead to an undesirable outcome, i.e., occurrence, progression, or recurrence of CRC. A human may be classified as "high risk" or "low risk" according to the data obtained from said human, sample or event. As to the risk score described in the present disclosure, the patient with a calculated risk score ≥ a predetermined risk score (e.g., 0.2, such as ≥ 0.211, ≥ 0.238, ≥ 0.387, or ≥ 0.458, depending on the biomarkers chosen) is classified as "high risk", which indicates that he/she have a higher probability of developing CRC within about five years than the other humans investigated. The patient with the calculated risk score < the predetermined risk score (e.g., 0.2, such as < 0.211, < 0.238, < 0.387, or < 0.458, depending on the biomarkers chosen) is classified as "low risk", which indicates that he/she have a lower probability of developing CRC within about five years than the other humans investigated.

As used herein, the term "prophylactic treatment" or "preventive treatment" are interchangeable, and refers to either preventing or inhibiting the development of a clinical condition or disorder or delaying the onset of a pre-clinically evident stage of a clinical condition or disorder; for example, CRC. According to embodiments of the present disclosure, the term "prophylactic treatment" refers to a preventative treatment for a human predisposed to CRC. In general, the predisposition may be due to genetic factors, age, sex, injury, and the like.

As used herein, the term "therapeutic treatment" refers to administering treatment to a human already suffering from a disease (e.g., CRC) thus causing a therapeutically beneficial effect, such as ameliorating existing symptoms, ameliorating the underlying metabolic causes of symptoms, postponing or preventing the further development of a disorder and/or reducing the severity of symptoms that will or are expected to develop.

The term "human" or "patient" refers to the human species that is evaluable with the method of the present disclosure. The term "human" or "patient" intended to refer to both the male and female gender unless one gender is specifically indicated, and may be at any age, e.g., a child or adult.

The term "amount," "concentration," or "level" of a target protein or a protein in an analyte means the physical quantity of the substance referred to, either in terms of mass (or equivalently moles) or in terms of concentration (the amount of mass or moles per volume of a solution or liquid sample). Also, the term "analyte" refers to a molecule, a portion, a piece, a fragment, or a section of a molecule, or a component released from cells comprising the same (e.g., an extracellular vesicle) that is to be measured or quantitated in a sample. An analyte may thus be, for example, a protein, a peptide derived from a protein by digestion or other fragmentation techniques, a small molecule (such as a hormone, a metabolite, a drug, a drug metabolite), a nucleic acid (DNA, RNA, or a fragment thereof produced by an enzymatic, a chemical, or other fragmentation processes), or a component released from cells comprising the same (e.g., an extracellular vesicle). The term "small molecule" or "metabolite" means a multi-atom molecule other than proteins, peptides and DNA; the term can include, but is not limited to, amino acids, steroid and other small hormones, metabolic intermediate compounds, drugs, drug metabolites, toxicants and their metabolites, and fragments of larger biomolecules.

As used herein, the term "extracellular vesicle (EV)" refers to all vesicles released from cells by any mechanism, therefore including secreted and exocytosed vesicles, thereby encompassing exosomes, but also including vesicles released by ectosytosis, reverse budding, fission of membrane(s) (as, for example, multivesicular endosomes, ectosomes, microvesicles and microparticles, etc.), and release of apoptotic bodies and hybrid vesicles containing acrosomal and sperm plasma membrane components. The EV is composed of a lipid bilayer composed of a cell membrane component, cell membrane lipids, membrane proteins, genetic material, and cytoplasmic components of the cell.

The term "mass spectrometer (MS)" means an instrument capable of separating molecules on the basis of their mass, m or m/z, where z is molecular charge, and then detecting them. In one embodiment, mass spectrometers detect molecules quantitatively. A MS may use one, two, or more stages of mass selection. In the case of multistage selection, some means of fragmenting the molecules is typically used between stages, so that later stages resolve fragments of molecules selected in earlier stages. Use of multiple stages typically affords improved overall specificity compared to a single stage device. Often, quantitation of molecules is performed in a triple-quadrupole mass spectrometer using the method referred to as "product ion scan (PIS)" mass spectrometry in which measured molecules are selected first by their intact mass and secondly, after fragmentation, by the mass of a specific expected molecular fragment. However, it will be understood herein that a variety of different MS configurations may be used to analyze the molecules described. Possible configurations include, but are not limited to, MALDI instruments including MALDI-TOF, MALDI-TOF/TOF, and MALDI-TQMS; electrospray instruments including ESI-TQMS and ESI-QTOF; and LC-based instruments including LC-MS, LC-MS/MS, and 2D-LC-MS/MS, in which TOF means time of flight, TQMS means triple quadrupole MS, ESI means electrospray ionization, QTOF means quadrupole TOF, LC means liquid chromatography, and 2D means two-dimensional.

The term "target peptide" means a peptide derived from a target protein which is chosen as a monitor fragment of the target protein.

The term "monitor fragment" refers to any portion of an analyte, but not including the whole analyte, that can be produced by a reproducible fragmentation process and whose abundance or concentration can be used as a surrogate for the abundance or concentration of the analyte.

The terms "stable isotope standard (SIS)" and "a stable isotope-labeled peptide or protein" mean a peptide or protein, such as a peptide or protein basing a unique sequence that is identical or substantially identical to that of a selected peptide or protein analyte, and including a label of some kind (e.g., a stable isotope) that allows its use as an internal standard for mass spectrometric quantitation of the natural (unlabeled, typically biologically generated) counterpart of the analyte. In one embodiment, a SIS peptide or protein comprises a peptide sequence that has a structure that is chemically identical to that of the molecule for which it will serve as a standard, except that it has isotopic labels at one or more positions that alter its mass. Accordingly, a SIS is: (i) recognized as equivalent to the analyte in a pre-analytical workflow, and is not appreciably differentially enriched or depleted compared to the analyte prior to mass spectrometric analysis; and (ii) differs from the analyte in a manner that can be distinguished by a mass spectrometer, either through direct measurement of molecular mass or through mass measurement of fragments (e.g., through MS/MS analysis), or by another equivalent means. Stable isotope standards include peptides having non-material modifications of this sequence, such as a single amino acid substitution (as may occur in natural genetic polymorphisms), substitutions (including covalent conjugations of cysteine or other specific residues), or chemical modifications (including glycosylation, phosphorylation, and other well-known post-translational modifications) that do not materially affect enrichment or depletion compared to the analyte prior to mass spectrometric analysis. Advantageously, a SIS contains a level of substitution of each stable isotope (e.g., C, N, or H) at the specific site or sites within the peptide structure where the isotope(s) is/are incorporated (i.e., those sites that depart significantly from the natural unenriched isotope distribution) of > 95%, > 96%, > 97%, or > 98%.

The term "stable isotope" means an isotope of an element naturally occurring or capable of substitution in proteins or peptides that is stable (does not decay by radioactive mechanisms) over a period of a day or more. The primary examples of interest in the context of the methods described herein are H, C, N, O, and S, of which the most commonly used are ²H, ¹³C, ¹⁵N, ¹⁸O, ³⁴S, or a combination thereof.

The term "standardized sample" means a protein or peptide sample to which one or more stable isotope-labeled one or more peptide or protein analytes have been added at known levels corresponding to test evaluation thresholds to serve as an internal standard or standards.

### II. Description of the Invention

The main purpose of the present disclosure is to provide a method that can detect a suspicious CRC in a human with high specificity and high sensitivity by using a panel of detector biomarkers via the method of mass spectrometry.

### 1. The diagnostic method

Accordingly, the first aspect of the present disclosure is directed to a method for determining whether a human has or is at risk of developing CRC by use of an an extracellular vesicle (EV) derived from an ex vivo biological sample isolated from the human. The method comprises the steps of:
(a) determining a concentration of (1) two target proteins of CD59 and TSPAN9 or (2) three target proteins of ADAM10, CD59, and TSPAN9 in the ex vivo biological sample by the steps of:
   (a-1) selecting two or three surrogate peptides corresponding to the two or three target proteins, wherein each of the two or three surrogate peptides is selected from the group consisting of ADAM10 surrogate peptide, CD59 surrogate peptide, and TSPAN9 surrogate peptide, wherein the ADAM10 surrogate peptide comprises the amino acid sequence of SEQ ID NO: 1; the CD59 surrogate peptide comprises the amino acid sequence of SEQ ID NO: 6; and the TSPAN9 surrogate peptide comprises the amino acid sequence of SEQ ID NO: 12;
   (a-2) labeling the two or three surrogate peptides of step (a-1) by isotopes;
   (a-3) digesting the EV derived from the ex vivo biological sample by means of a proteolytic process to produce a digest;
   (a-4) adding a predetermined concentration of the isotope-labeled two or three surrogate peptides of step (a-2) to the digest of step (a-3);
   (a-5) determining the amounts of the target peptides and the isotope-labeled two or three surrogate peptides in the mixture of step (a-4) by mass spectrometry;
   (a-6) dividing the determined amounts of the two or three target peptides by the determined amounts of the two or three isotope-labeled surrogate peptides to produce a ratio; and
   (a-7) determining the concentration of the two or three target proteins in the EV drerived from the ex vivo biological sample based on the ratio of step (a-6) and the predetermined concentration of the isotope-labeled surrogate peptides of step (a-4);
(b) calculating a risk score based on the concentrations of the two or three target proteins determined in step (a); and
(c) determining whether the human has or is at risk of developing CRC based on the calculated risk score of step (b), wherein the human does not have or is at low risk of developing CRC if the calculated risk score of step (b) is lower than a predetermined risk score, and the humanhas or is at high risk of developing CRC if the calculated risk score of step (b) is the same or above the predetermined risk score.

To start with, an extracellular vesicle (EV) derived from an ex vivo biological sample is obtained from a human. According to some embodiments of the present disclosure, the ex vivo biological sample is preferably blood, plasma, serum, saliva, sputum, urine, ascites, cerebrospinal fluid, amniotic fluid, or tissue lysate. The ex vivo biological sample comprises an EV therein.

The method for isolating an EV from a biological sample may be carried out according to a conventional method and is not particularly limited. Examples of the method for isolating an EV from a biological sample include an affinity method (e.g., a phosphatidylserine (PS) affinity method); a fractional centrifugation method (e.g., an ultracentrifugation method such as a pellet-down method, a sucrose cushion method, or a density gradient centrifugation method); an immunoprecipitation method; chromatography (e.g., ion exchange chromatography or gel permeation chromatography); a density gradient method (e.g., a sucrose density gradient method); electrophoresis (e.g., organelle electrophoresis); a magnetic separation method (e.g., magnetically activated cell sorting (MACS) method); an ultrafiltration concentration method (e.g., a nanomembrane ultracentrifugation concentration method); a Percoll gradient isolation method; a method using a microfluidic device; and a PEG precipitation method. An affinity method with which an EV having a high degree of purity can be obtained, or a fractional centrifugation method that enables theoretically unbiased recovery is preferable, an affinity method or an ultracentrifugation method is more preferable. One of these isolation methods may be used alone, or two or more methods may be combined. In addition, isolation by one isolation method may be repeated twice or more. The examination of the isolated EV may be followed through with any methods known in the art, for example, transmission electron microscopy, nanoparticle tracking analysis (NTA), flow cytometric analysis, western blot analysis to detect markers predominantly expressed on EVs, or a combination thereof.

In step (a), the levels of the two or three target proteins of CD59 and TSPAN9 or ADAM10, CD50 and TSPAN9 in the sample are determined by performing the following steps (a-1)-(a-7). According to some embodiments of the present disclosure, two target proteins of ADAM10, CD59, and TSPAN9 are quantified (either as relative values or absolute values) so as to produce a two-marker panel useful in making a diagnosis or a prognosis of the CRC. Such a two-marker panel may be: (1) ADAM10 and CD59 polypeptides, (2) CD59 and TSPAN9 polypeptides, or (3) TSPAN9 and ADAM10 polypeptides. According to other embodiments of the present disclosure, all target proteins of ADAM10, CD59, and TSPAN9 are quantified (either as relative values or absolute values) so that a three-marker panel is produced.

In general, the levels of ADAM10, CD59, and/or TSPAN9 can be determined by any assay familiar with the skilled artisan; for example, ELISA, strip-based rapid test, western blotting, mass spectrometry, protein microarray, flow cytometry, immunofluorescence, immunohistochemistry, multiplex detection assay, and nuclear magnetic resonance. According to some preferred embodiments of the present disclosure, the levels of ADAM10, CD59, and/or TSPAN9 is determined by LC-MS/MS with product ion scan (PIS) mode (LC-PIS-MS), an assay widely used in the field of proteomics that provides a specific and precise quantification of the polypeptides of interests.

In step (a-1) two or three surrogate peptides corresponding to the two or three target proteins are selected, in which the two or three surrogate peptides may be corresponding to any fragments of their natural counterpart with the amino acid sequence thereof having at least 90% identity to their natural counterpart, in which the fragment may be 5-25 amino acids in length; preferably, 8-20 amino acids in length; more preferably, 7-15 amino acids in length. That is, the surrogate peptide of ADAM10 comprises an amino acid sequence at least 90% (i.e., 90%, 91%, 92%, 93%, 94%, 95%, 96%, 97%, 98%, 99%, or 100%) identical to any fragments of SEQ ID NO: 15; the surrogate peptide of CD59 comprises an amino acid sequence at least 90% (i.e., 90%, 91%, 92%, 93%, 94%, 95%, 96%, 97%, 98%, 99%, or 100%) identical to any fragments of SEQ ID NO: 16; and the surrogate peptide of ADAM10 comprises an amino acid sequence at least 90% (i.e., 90%, 91%, 92%, 93%, 94%, 95%, 96%, 97%, 98%, 99%, or 100%) identical to any fragments of SEQ ID NO: 17.

According to the present disclosure, the fragment within the natural counterpart is predetermined; and such fragment is SEQ ID NO: 1 of ADAM10, SEQ ID NO: 6 of CD59, or SEQ ID NO: 12 of TSPAN9. In such case, the surrogate peptide of ADAM10 comprises an amino acid sequence at least 90% (i.e., 90%, 91%, 92%, 93%, 94%, 95%, 96%, 97%, 98%, 99%, or 100%) identical to SEQ ID NO: 1; the surrogate peptide of CD59 comprises an amino acid sequence at least 90% (i.e., 90%, 91%, 92%, 93%, 94%, 95%, 96%, 97%, 98%, 99%, or 100%) identical to SEQ ID NO: 6; and the surrogate peptide of TSPAN9 comprises an amino acid sequence at least 90% (i.e., 90%, 91%, 92%, 93%, 94%, 95%, 96%, 97%, 98%, 99%, or 100%) identical to SEQ ID NO: 12. According to the working example of the present disclosure, the surrogate peptide of ADAM10 has the amino acid sequence of SEQ ID NO: 1; the surrogate peptide of CD59 has the amino acid sequence of SEQ ID NO: 6; and the surrogate peptide of TSPAN9 has the amino acid sequence of SEQ ID NO: 12.

In step (a-2), the two or three surrogate peptides of step (a-1) are labeled with isotopes, in particular, stable isotopes (e.g., ²H, ¹³C, ¹⁵N, ¹⁸O, and/or ³⁴S) (hereafter, "the SIS peptide"). The method for isotope labeling is well known in the art, and the surrogate peptide may be labeled with a specific amino acid with a specific isotope, for example, the surrogate peptide may be labeled with ¹³C/¹⁵N-labeld Lys and Arg, which may be commercially available. Thus, the SIS peptide is the same as the target protein, but for the presence of the stable isotope label.

Next, the EV derived from the ex vivo biological sample is digested to produce a digest (the step (a-3)); the digestion may be achieved chemically or enzymatically, in which the chemical digestion is carried out by applying cyanogen bromide (CNBr), CNBr/formic acid or CNBr/70% formic acid to the sample, and the enzymatical digestion is carried out by applying trypsin, chymotrypsin, LysN, LysC, Glu-C, Asp-N, ArgC, pepsin, proteinase K, elastase, thermolysin, papain, subtilisin, or combinations thereof to the sample. According to preferred embodiments of the present disclosure, the ex vivo biological sample is enzymatically digested.

For determining the level of the target proteins, first, a predetermined concentration of the SIS peptides of step (a-2) is added to the digest of step (a-3) (the step (a-4)). A detection instrument, such as a mass spectrometer (e.g., LC-MS/MS), is used to detect or measure the presence of the SIS peptides and the target proteins in the resulting mixture of step (a-4) (hereafter, "the test sample") (the step (a-5)). An instrument signal magnitude from the detection instrument is measured for the SIS peptides and the target proteins. Exemplary instrument signal magnitudes include intensity, counts, area under a curve, or combinations thereof. Quantification of the at least two target proteins is achieved by plotting or generating the ratio of the instrument signal magnitude (i.e., the peak area (the area under the curve) of the target protein to the instrument signal magnitude of the SIS peptide in the test sample, and aligning the resultant ratio with the best fit line and interpolating or extrapolating the concentration of the target protein in the test sample, e.g., via the calculation set forth in steps (a-6)-(a-7). The concentration of the at least two target proteins is thus determined.

Alternatively or in addition, a calibration curve may be generated to assist in the precise quantification of the concentration of the target protein in the test sample, by comparing the ratio of the SIS peptide in the test sample (where the SIS peptide is served as an internal standard; hereafter, "the internal SIS peptide") with the ratio of the SIS peptide in the calibration curve (hereafter, "the calibration SIS peptide"), and then through calculation (either interpolation or extrapolation) to obtain the precise concentration of the target protein. The calibration curve may be generated from a control sample, using the calibration SIS peptide at a series of known concentrations and plotting the ratios against the known concentrations of the calibration SIS peptide. The calibrations can be used in a number of different concentrations, e.g., 2, 3, 4, 5, 6, 7, 8, 9, 10, 11, 12, 13, 14, 15 or more different concentrations. The calibration concentrations can be serial dilutions, for example, the concentrations may differ by a factor of 2, 3, 4, 5, 6, 7, 8, 9, 10, or more. The calibration concentrations should span a suspected concentration of target protein. The internal SIS peptide is used in a uniform concentration among each of the calibration SIS peptide containing control samples, the test sample, and any quality control sample. The generated calibration plot is fit with a best fit line. The best fit may be linear or curved.

In step (b), the panels of the two- or three-markers quantified in step (a) are used to calculate the predictive probability as a risk score. According to some embodiments of the present disclosure, the risk score is calculated based on the analysis of the panel of the two-markers, which comprises two target proteins selected of CD59, and TSPAN9. According to other embodiments of the present disclosure, the risk score is calculated based on the analysis of the panel of the three-markers, which comprises three target proteins of ADAM10, CD59, and TSPAN9. According to some embodiments of the present disclosure, logistic regression is used to analyze the panels of the two- or three-markers in the purpose of calculating the risk score. According to preferred embodiments of the present disclosure, the risk score is calculated using an equation of: $risk score = \frac{{e}^{\left(a+b1X1+b2X2+b3X3\right)}}{1 + {e}^{\left(a+b1X1+b2X2+b3X3\right)}}$ wherein e is a mathematical constant that is the base of the natural logarithm; a is a constant value; X1, X2, and X3 respectively represent the concentrations of ADAM10, CD59, and TSPAN9; and b1, b2, and b3 respectively represent the coefficient of variation of ADAM10, CD59, and TSPAN9.

According to one working example of the present disclosure, the constant value and the coefficient of variation may vary with the marker panel, and the predetermined risk score established by specified target proteins is calculated in accordance with the equations listed in the paragraphs [0112] and [0115] and is provided in Tables 6-7.

In step (c), the risk score calculated in step (b) is used to assess whether the human has or is at risk of developing CRC. According to some embodiments of the present disclosure, the predetermined risk score as described above is useful in distinguishing non-CRC human (i.e., healthy human) from CRC or early-stage (stage I-II) CRC patients. In these embodiments, when the calculated risk score of step (b) is lower than the predetermined risk score (e.g., < 0.2), it is an indicia that the human does not have CRC; when the calculated risk score of step (b) equals to or is higher than the predetermined risk score (e.g., ≥ 0.2), it is an indicia that the human has CRC (with the sensitivity of 89.04-98.63% and the specificity of 75-92.5%) or has early-stage (stage I-II) CRC (with the sensitivity of 96.88-100% and the specificity of 75-92.5%). Alternatively or in addition, the predetermined risk score varies with the at least two target proteins chosen.

According to some embodiments of the present disclosure, the human does not have CRC or is at low risk of developing CRC if any of the following condition prevails:
(1) the two target proteins of step (a) are CD59 and TSPAN9, and the calculated risk score of step (b) is lower than the predetermined risk score of step (c) (i.e., 0.211), or
(2) the three target proteins of step (a) are ADAM10, CD59, and TSPAN9, and the calculated risk score of step (b) is lower than the predetermined risk score of step (c) (i.e., 0.238).

According to other embodiments of the present disclosure, the human has CRC or is at high risk of developing CRC if any of the following conditions prevails:
(1) the two target proteins of the step (a) are CD59 and TSPAN9, and the calculated risk score of step (b) is the same or above the predetermined risk score of step (c) (i.e., 0.211), or
(2) the three target proteins of the step (a) are ADAM10, CD59, and TSPAN9, and the calculated risk score of step (b) is the same or above the predetermined risk score of step (c) (i.e., 0.238).

The clinical practitioner may make a prompt diagnosis and treatment to the human in need thereof in accordance with the present risk score derived from the present method, in which the human having the calculated risk score of step (b) equal to or higher than the predetermined risk score (e.g., ≥ 0.2), as described above, shall be subjected to an anti-cancer treatment (e.g., a prophylactic treatment or a therapeutic treatment) or be placed in an intensive follow-up regimen.

### 2. The treatment method

The present disclosure also describes a method for diagnosing and treating CRC in a human. The method comprises determining whether or not a human has CRC by the steps (a) to (c) in the foregoing method followed by administering an effective amount of an anti-cancer treatment to the human having the calculated risk score of step (b) equal to or higher than the predetermined risk score (e.g., ≥ 0.2), as described above. In general, the anti-cancer treatment may be a preventive treatment (e.g., a surgery such as endoscopic submucosal dissection), a therapeutic treatment (e.g., a surgery, a chemotherapy, a radiotherapy, an immunotherapy, a targeted therapy, or a thermotherapy therapy), or a combination thereof.

Said chemotherapy is achieved by administering a chemotherapeutic agent to the human, such chemotherapeutic agent may be actinomycin D, aminoglutethimide, amsacrin, anastrozol, anthracycline, bexaroten, bleomycin, buselerin, busulfan, camptothecin derivates, capecitabin, carboplatin, carmustine, chlorambucil, cisplatin, cladribin, cyclophosphamide, cytarabin, cytosinarabinoside, dacarbacin, dactinomycin, daunorubicin, docetaxel, doxorubicin, epirubicin, estramustine, etoposid, exemestan, fludarabin, fluorouracil, formestan, gemcitabin, goselerin, hycamtin, idarubicin, ifosfamid, imatinib, irinotecan, letrozol, leuprorelin, lomustin, melphalan, mercaptopurine, methotrexate, miltefosin, mitomycine, mitoxantron, nimustine, oxaliplatin, paclitaxel, pentostatin, procarbacin, temozolomid, teniposid, testolacton, thiotepa, thioguanine, topotecan, treosulfan, tretinoin, triptorelin, trofosfamide, vinblastine, vincristine, vindesine, or vinorelbine.

The immunotherapy is achieved by administering an immunotherapeutic agent to the human. Non-limiting examples of the immunotherapeutic agent include, but are not limited to, an anti-PD-1 antibody (e.g., pembrolizumab or nivolumab), an anti-PD-L1 antibody (e.g., atezolizumab, avelumab, or durvalumab), an anti-CTLA-4 antibody (e.g., ipilimumab or tremelimumab), riluzole, trigriluzole, IFN-y, IL-2, IL-15, IL-23, M-CSF, GM-CSF, TNF, CD80, CD86, and ICAM-1.

The targeted therapy is achieved by administering a targeted agent to the human. examples of the targeted agent include, but are not limited to, bortezomib, dasatinib, erlotinib, gefitinib, lapatinib, nilotinib, sorafenib, sunitinib, tofacitinib, crizotinib, venetoclax, obatoclax, navitoclax, gossypol, olaparib, rucaparib, niraparib, talazoparib, perifosine, apatinib, vemurafenib, dabrafenib, trametinib, vismodegib, sonidegib, salinomycin, vintafolide, temsirolimus, everolimus, rituximab, trastuzumab, alemtuzumab, cetuximab, panitumumab, bevacizumab, and ipilimumab.

### 3. The pharmaceutical kit

Also described herein is a pharmaceutical kit for determining whether a human has or is at risk of developing CRC. The present pharmaceutical kit comprises at least two agents (e.g., two or three agents) for use in determining the levels of at least two of ADAM10, CD59, and TSPAN9 (i.e., any two or three of ADAM10, CD59, and TSPAN9) in the human. For example, the present pharmaceutical kit may comprise two agents respectively useful for quantifying the levels of any two of ADAM10, CD59, and TSPAN9. Alternatively, the present pharmaceutical kit may comprise three agents respectively useful for quantifying the levels of ADAM10, CD59, and TSPAN9. According to one working example of the present disclosure, each of the agents is an isotope-labeled polypeptide, in which the agents for quantifying ADAM10 (SEQ ID NO: 15), CD59 (SEQ ID NO: 16), and TSPAN9 (SEQ ID NO: 17) respectively comprise the amino acid sequences of SEQ ID NOs: 1, 6, and 12.

The assay for determining the levels of ADAM10, CD59, and/or TSPAN9 may be carried out with LC-MS/MS. The quantified values of ADAM10, CD59, and/or TSPAN9 are then used to calculate a risk score so as to make a diagnosis or risk evaluation of CRC. As mentioned above, the risk score may be calculated by use of logistic regression; preferably, by an equation of: $risk score = \frac{{e}^{\left(a+b1X1+b2X2+b3X3\right)}}{1 + {e}^{\left(a+b1X1+b2X2+b3X3\right)}}$ wherein e is a mathematical constant that is the base of the natural logarithm; a is a constant value; X1, X2, and X3 respectively represent the concentrations of ADAM10, CD59, and TSPAN9; and b1, b2, and b3 respectively represent the coefficient of variation of ADAM10, CD59, and TSPAN9.

According to one working example of the present disclosure, the constant value and the coefficient of variation may vary with the marker panel, and the predetermined risk score established by specified target proteins is calculated in accordance with the equations listed in the paragraphs [0112] and [0115] and is provided in Tables 6-7.

According to embodiments of the present disclosure, in accordance with the present method, when the calculated risk score of step (b) is lower than the predetermined risk score (e.g., < 0.2), then the human does not have CRC or is at low risk of developing CRC; and when the calculated risk score of step (b) is the same or above the predetermined risk score (e.g., ≥ 0.2), then the human has CRC or is at high risk of developing CRC. Alternatively, the predetermined risk score is dependent on the at least two target proteins chosen, as described above; for the sake of brevity, the description is omitted herein.

### EXAMPLES

### Materials and Methods

### 1. Clinical specimens

Peripheral blood samples were collected from healthy donors and histologically confirmed, treatment naive CRC patients at the Department of Colon and Rectal Surgery, Chang Gung Memorial Hospital from 2016 to 2020 (New Taipei city, Taiwan) (Table 1). Plasma samples were prepared from the peripheral blood samples by centrifugation at 2,000 ×g for 10 minutes at room temperature. The supernatants, i.e., the plasma samples, were divided into 1.0 ml aliquots and immediately frozen at -80°C for later use. To generate the panel of the biomarkers used in the present study for screening CRC, a set of cohorts (HC, n = 30; CRC, n = 30) were used in the discovery stage (hereafter, "the training set"), to explore potential candidates for serving as a biomarker. After selection of the potential candidates, a set of cohorts (HC, n = 80; CRC, n = 73) were used in the verification stage (hereafter, "the test set"), to verify the ability of the potential candidates for serving as a biomarker. The verification was done by MS quantification with the product ion scan (PIS) mode. The plasma samples derived from all the healthy humans and the CRC patients were included in the experiments, and EVs were isolated individually from those cases, including both the healthy control and the CRC cases. All CRC patients had histologically confirmed adenocarcinoma. The patients' characteristics were gathered from clinical and pathological records including gender, age, tumor location, histological grade, tumor stage, CEA level, preoperative laboratory data, operation date, operation method, tumor recurrence, follow-up date, and follow-up status. All patients were subjected to routine checkups following the standard medical protocol. All the individuals submitted the informed consent form for blood donation according to the rules and regulation approved by the institutional review board (IRB No. 201601848B0 and 201801888B0).

**Table 1 Clinicopathological characteristics of the humans from whom plasma samples were obtained and used in this study**

| | | The training set (n=60) | | The test set (n=153) | |
|---|---|---|---|---|---|
| Characteristics | | HC | CRC | HC | CRC |
| Gender | Female | 13 | 10 | 36 | 37 |
| | Male | 17 | 20 | 44 | 36 |
| Age (years)^{a} | - | 57.43 ± 10.76 | 60.40 ± 10.70 | 58.51 ± 7.79 | 57.77 ± 4.69 |
| Tumor stage (T) | T1 | - | 2 | - | 14 |
| | T2 | - | 3 | - | 9 |
| | T3 | - | 18 | - | 42 |
| | T4 | - | 7 | - | 8 |
| Lymph node metastasis (N) | No | - | 13 | - | 37 |
| | Yes | - | 17 | - | 36 |
| Distant metastasis (M) | No | - | 23 | - | 57 |
| | Yes | - | 7 | - | 16 |
| TNM stage | I | - | 3 | - | 18 |
| | II | - | 9 | - | 14 |
| | III | - | 11 | - | 25 |
| | IV | - | 7 | - | 16 |

| | | | | | |
|---|---|---|---|---|---|
| ^{a} Data are shown in mean ± standard deviation (S.D.). | | | | | |

### 2. EV isolation from the plasma samples

The plasma samples (1 ml per case) were diluted 10-fold with PBS and centrifuged sequentially at 500 ×g for 5 minutes and 2,000 ×g for 15 minutes. The supernatants were transferred to ultracentrifuge tubes and centrifuged at 10,000 ×g for 30 minutes. Then, the supernatants were collected and centrifuged at 110,000 ×g for 2 hours. After centrifugation, the supernatants were discarded, and the residual pellets were dissolved in PBS, filtered through a 0.22 µm filter, and centrifuged again at 110,000 ×g for 90 minutes. The resulting pellets were resuspended in 200 µl PBS and stored at -80°C. All the centrifugation procedures were performed at 4°C.

### 3. Selection and synthesis of surrogate peptides for potential targets

One surrogate peptide was selected for each potential target. To this end, all the potential targets were subjected to in-silico tryptic digestion using the online MS digest software, and surrogate peptides for each potential target were selected following the criteria: (a) unique peptides containing 8-20 residues without any known post-translational modification sites, which is determined from the human protein reference database and no sequential or missed trypsin cleavage sites; (b) peptides without chemically reactive amino acids, such as C, M, and W; (c) peptides without unstable sequences, such as NG, DG, and QG; and (d) peptides without sequences potentially leading to missed cleavage, such as RP and KP. The selected surrogate peptides for the 13 potential candidates and CD9, an EV marker and to serve as a control marker, and their sequence were summarized in Table 2. After the surrogate peptides were selected, the surrogate peptides were synthesized and labeled with stable isotopes, ¹³C/¹⁵N on Lys and Arg (the SIS peptides or synthetic heavy peptides; served as internal standards herein), thereby making 8 and 10 Da heavier for the SIS peptides harboring Lys and Arg (purchased from Thermo Fisher Scientific, USA). The purity of the SIS peptides is higher than 95% (most of their purity is higher than 98%), as verified by mass spectrometry analysis after HPLC purification. Surrogate peptides without stable isotope labeling (synthetic light peptides) were also synthesized (purchased from Kelowna International Scientific).

**Table 2 Surrogate peptides for potential targets used in the present study**

| **SEQ ID NO** | **Gene name** | **Sequence of surrogate peptides** | **Precursor m/z** | |
|---|---|---|---|---|
| | | | **Heavy** | **Light** |
| 1 | ADAM10 | AIDTIYQTTDFSGIR | 855.932 | 850.928 |
| 2 | ALCAM | VLHPLEGAVVIIFK | 514.988 | 512.316 |
| 3 | APMAP | GLFEVNPWK | 549.297 | 545.29 |
| 4 | ART4 | FGQFLSTSLLK | 624.857 | 620.850 |
| 5 | CD58 | VAELENSEFR | 602.297 | 597.293 |
| 6 | CD59 | AGLQVYNK | 450.755 | 446.748 |
| 7 | CD9 | EVQEFYK | 475.739 | 471.732 |
| 8 | ICAM3 | IALETSLSK | 485.289 | 481.281 |
| 9 | ITGAM | LFTALFPFEK | 610.843 | 606.836 |
| 10 | RHAG | FLTPLFTTK | 538.317 | 534.310 |
| 11 | SELP | NEIDYLNK | 508.760 | 504.753 |
| 12 | TSPAN9 | EGLLLYHTENNVGLK | 854.461 | 850.454 |
| 13 | TSPAN33 | DDLDLQNLIDFGQK | 821.414 | 817.407 |
| 14 | TTYH3 | VLHPLEGAVVIIFK | 768.327 | 763.323 |

### 4. Preparation of EV samples for MS-based targeted protein quantification

Five µg of the previously isolated EVs was dissolved in PBS containing 100 mM Tris buffer at pH 8.5, reduced by 5 mM Tris (2-carboxyethyl) phosphine (TCEP) at 60°C for 30 minutes, and then alkylated by 10 mM iodoacetamide (IAA) in dark for 30 minutes at room temperature. The EV samples were diluted 2-fold with 100 mM Tris, and digested by 0.2 µg trypsin at 37°C for 16 hours. A standard cocktail containing the above 14 SIS peptides (50 fmole per peptide) was then added into each of the EV samples, and 0.5% formic acid (FA) and 0.2% trifluoroacetic acid (TFA) were used to acidify the EV samples and stop the trypsin reaction. The digested EV samples were desalted with solid-phase extraction Oasis HLB (30 µm) cartridges (Waters, MA), and then lyophilized for later quantitative MS analysis with the product ion scan mode.

### 5. LC-PIS-MS analysis and data processing

Dried peptides (1 µg) were reconstituted in 0.1% FA, loaded across a trap column (Zorbax 300SB-C18 5 µm, 0.3 × 5 mm; Agilent Technologies) at a flow rate of 20 µl/min in HPLC mobile phase A, and resolved on an analytical column (ACQUITY UPLC C18 column 1.7 µm, 0.1 × 100 mm) with HPLC mobile phase B (100% acetonitrile (ACN)/0.1% FA) at a flow rate of 0.4 µl/min. The samples (containing the 14 target peptides) were separated with a 11.3-minute linear gradient of 8-35% HPLC mobile phase B, and with two-steps of 95% HPLC mobile phase B in sequence, so as to prevent sample carryover on the analytical column. The eluted peptides were analyzed with a two-dimensional linear ion trap mass spectrometer LTQ-Orbitrap ELITE. Intact peptides were detected in the Orbitrap at a resolution of 120,000. Internal calibration was performed using the ion signal of (Si(CH₃)₂O)₆H⁺ at m/z of 536.165365 as a lock mass. For MS analysis with the PIS mode, accurate m/z values of precursor ions and their retention times were initially imported into the inclusion list. The MS/MS analysis was performed on each targeted precursor, and the generated product ions were detected in the linear trap quadrupole (LTQ) followed the scheduled scanning times. The automatic gain control (AGC) value of MS and MS/MS were set to 3 × 10⁶ ions (full scan) at 1000 milliseconds (ms) and 5× 10³ (collision-induced dissociation (CID)) at 300 ms for maximum accumulated time or ions, respectively.

All data (the RAW files of the spectra) obtained from the LTQ-Orbitrap ELITE were processed using the SEQUEST algorithm to search against the peptide sequences derived from the 14 potential targets, and the search results were integrated by Proteome Discoverer 1.4 (PD1.4; Thermo Fisher, CA). Analysis was performed using the following parameters: the MS tolerance for the monoisotopic peptide window was set to 10 ppm; the MS/MS tolerance was set to 0.6 Da; the dynamic modification was set with stable isotope-containing lysine (+8 Da) and arginine (+10 Da); the charge state of the peptides were set to 2⁺ and 3⁺; the false discovery rate (FDR), calculated by peptide sequence analysis to distinguish true positives from random matches (decoy database), was set to 1% as a cut-off threshold for ensuring confidence of peptide identification. Spectral libraries of sample runs, constructed from the PD1.4 software in msf format, and the relevant RAW files were imported into the Skyline software (v. 21.1.0.278). For analyzing the MS spectra from the PIS mode, product ions with the ten highest values of intensity were automatically selected by Skyline from ion 1 to last ion with 1⁺, 2⁺, and 3⁺ ion charges in the 300-1250 m/z range, and the ion match tolerance was set to 0.7 m/z. After manually removing ions with interference and checking their position of retention time, the integral area of trapezoidal model with unsmoothed chromatogram was applied to determine the final peak area. The specific peaks of the endogenous peptides (derived from the natural target proteins) were recognized according to their co-elution with the exogenously supplemented SIS peptides, and then the peak area of 2 to 7 selected fragments of target peptides were summarized for further quantitative analysis. The concentration of the target peptides in the samples was gained from calculating the ratio of the peak area of the target peptides to that of the corresponding SIS peptides at a known-concentration; the concentration of the target peptides represented the concentration of the corresponding target proteins. Three independent experimental replicates were performed (from digestion to the final LC-PIS-MS step) for each of the plasma EV samples.

### 6. Generation of calibration curves

Calibration curves for the 14 SIS peptides were generated from quintuplicate experiments. For each target, a series of the serially diluted heavy peptides at the amounts of 0, 0.049, 0.098, 0.195, 0.391, 0.781, 1.563, 3.125, 6.25, 12.5, 25, 50, and 100 fmol, and the light peptide at the constant amount 10 fmol, were spiked into an EV-protein digest background (1 µg protein) and analyzed by LC-PIS-MS. A MRM statistical software, QuaSAR, was used to assess the limit of detection (LOD) for each target by applying the method of "blank and low concentration sample" to estimate the mean of the blank samples and the standard deviation (S.D.) of the blank and low-concentration samples. The lower limit of quantification (LLOQ) was set as the LOD value multiplied by 3.

### 7. Statistical Analysis

Data were presented as mean ±S.D.. The Mann-Whitney test was used to compare the differences in the level of the selected proteins in plasma derived EVs between the HC and CRC groups. The Mann-Whitney test and the Kruskal-Wallis test were used to evaluate the association of the level of ADAM10, CD59, TSPAN9, and CEA in the plasma-EVs with various clinicopathological parameters of CRC patients. The diagnostic power of ADAM10, CD59, TSPAN9, and CEA was analyzed by constructing a receiver operating characteristic (ROC) curve with sensitivity versus 1-specificity, and calculating area under the ROC curve (AUC). For all statistical analyses, a two-tailed p-value ≤ 0.05 was considered significant. Calculations and diagrams were generated using the software GraphPad Prism 7.0 (GraphPad Software, CA).

### Example 1 Selection of the potential candidates as biomarkers for CRC

The discovery of searching potential candidates as biomarkers was in accordance with an internal, undisclosed high-throughput research, in which the presence of the membrane protein targets on EV in the clinical plasma EV specimens was determined via AIMS analysis (dataset 1) and iTRAQ-based proteomics analysis (dataset 2), using the pooled plasma EV samples isolated from 30 healthy controls and those isolated from 30 CRC patients (i.e., the training set) (data not shown). Potential candidates of biomarker were selected by integrated analysis of these two datasets, specifically, based on the AIMS and iTRAQ results, 58 potential candidates were preliminarily selected to be verified by the fold change between HC and CRC and by their expression levels in CRC, in which the 58 potential candidates were categorized into three groups: (1) tier 1: including all the membrane proteins including cell surface, peripheral, and integral proteins (n = 25); (2) tier 2: including all the membrane associated proteins (n = 20); and (3) tier 3: including other unclassified proteins (n = 13). As per the belief that the membrane proteins can be easily accessed in the plasma EVs, the 25 potential candidates pertaining to the tier 1 group were subjected to further verification. The expression of the 25 potential candidates in the tier 1 group in EVs isolated from individual plasma samples from 5 healthy controls and from 6 CRC patients was checked by AIMS (data not shown), and final 13 potential candidates (including ADAM10, ALCAM, APMAP, ART4, CD58, CD59, ICAM3, ITGAM, RHAG, SELP, TSPAN9, TSPAN33, and TTYH3; Table 2) were selected based on criteria that: (1) the fold change of CRC to HC was greater than 1.5; and (2) there should be more than 1 case detectable in the CRC group for further verification by MS-based, targeted protein quantification. The 13 potential candidates and CD9 (an EV marker and a control marker) were subjected to verification in 153 individual plasma EV samples isolated from 80 healthy donors and 73 CRC patients (i.e., the test set), by targeted MS analysis with the PIS mode.

### Example 2 Verification of the surrogate peptides of the selected potential candidates

In the present example, an experiment setup for employing a LC-MS/MS assay with the PIS mode (LC-PIS-MS) was established, that is to include all the surrogate peptides representing the above 14 targets (including synthetic light and heavy peptides for each candidate, 28 surrogate peptides in total; Table 2) in a LC-PIS-MS assay setup, to form a multiplex LC-PIS-MS assay setup able to measure the above 14 targets in a single run. Next, the performance of the above surrogate peptides in the multiplex LC-PIS-MS setup for measuring the above 14 targets was assessed, by creating calibration curves for the above surrogate peptides in measuring the above 14 targets, by LC-PIS-MS analysis. Calibration curves were created in accordance with the results of analyzing the response of the surrogate peptides, including a series of the serially diluted synthetic heavy peptides (0.488-100 fmol, 12 data points in total) mixing with a constant amount of the synthetic light peptide (10 fmol) for each target, in a background of plasma EV digest (containing 1 µg protein). The details of the calibration curves of all the 14 surrogate peptides, including the linearity of the response curves, the values for limit of detection (LOD) and for the lower limit of quantification (LLOQ), and the coefficient of variation (CV) for all 14 proteins are summarized in Table 3. Thirteen of the 14 targets (13/14, 93%) were detected in more than 9 points on the 12-point response curve (blank plus 12 points of 2-fold serial dilutions) with a good linear response (R² > 0.97). Eleven targets showed LLOQ values for peptides less than 1 fmol/µg, in which three targets (ALCAM, CD9, and TSPAN9) had higher LLOQ values ranging from 1.072 to 1.943 fmol/µg. The LLOQ value for each target is also expressed as protein concentration (in ng/ml), which represents the amount of the target protein corresponding to the determined level of the target peptide assuming complete tryptic digestion (i.e., 100% recovery), based on total protein concentration (0.0387 µg/µl) of the background EV protein digest. Eight targets (ADAM10, APMAP, ART4, CD58, CD59, ICAM3, RHAG, and TSPAN33) had LLOQ values less than 1 ng/ml protein. The remaining six targets (ALCAM, CD9, ITGAM, SELP, TSPAN9, and TTYH3) had LLOQ values between 1 and 5 ng/ml. The median value of CV, calculated in the linear range of the response curve for each target, was less than 12% for all the 14 proteins, indicating high stability in the assay (Table 3). Collectively, these data indicated that the present surrogate peptides in the multiplexed LC-PIS-MS assay show good stability and linearity, and target-dependent LLOQ in plasma EV samples containing 1 µg protein, in which a majority of targets exhibited LLOQ values for less than 1 fmol/µg (or 2 ng/ml).

**Table 3 Summary of precision and performance of the 14-plex LC-PIS-MS assay**

| **Protein** | **Linearity of response curve** | | | | | **LOD** | | **LLOQ** | |
|---|---|---|---|---|---|---|---|---|---|
| | **Slope** | **R square** | **y-intercept** | **Data points** | **Median CV%^{a}** | **(fmol/mg)** | **(ng/ml)^{b}** | **(fmol/mg)** | **(ng/ml)^{b}** |
| ADAM10 | 0.926 | 0.982 | 0.004 | 12 | 11.623 | 0.072 | 0.234 | 0.216 | 0.702 |
| ALCAM | 0.461 | 0.987 | 0.045 | 9 | 10.407 | 0.648 | 1.631 | 1.943 | 4.893 |
| APMAP | 0.949 | 0.991 | 0.005 | 12 | 7.949 | 0.081 | 0.146 | 0.244 | 0.439 |
| ART4 | 1.044 | 0.984 | 0.004 | 12 | 6.376 | 0.071 | 0.098 | 0.212 | 0.295 |
| CD58 | 1.149 | 0.984 | 0.002 | 12 | 9.628 | 0.059 | 0.064 | 0.177 | 0.192 |
| CD59 | 0.981 | 0.995 | 0.024 | 12 | 7.118 | 0.235 | 0.129 | 0.704 | 0.386 |
| CD9 | 1.002 | 0.986 | 0.021 | 10 | 9.265 | 0.48 | 0.472 | 1.44 | 1.415 |
| ICAM3 | 0.986 | 0.972 | 0.004 | 12 | 11.235 | 0.075 | 0.173 | 0.225 | 0.518 |
| ITGAM | 0.865 | 0.968 | 0.003 | 12 | 8.415 | 0.081 | 0.399 | 0.243 | 1.196 |
| RHAG | 1.047 | 0.987 | 0.004 | 12 | 8.195 | 0.097 | 0.166 | 0.292 | 0.499 |
| SELP | 1.073 | 0.993 | 0.009 | 11 | 9.594 | 0.203 | 0.714 | 0.61 | 2.142 |
| TSPAN33 | 0.671 | 0.98 | 0.075 | 12 | 9.438 | 0.08 | 0.098 | 0.241 | 0.294 |
| TSPAN9 | 1.042 | 0.995 | 0.02 | 10 | 5.978 | 0.357 | 0.37 | 1.072 | 1.11 |
| TTYH3 | 0.843 | 0.993 | 0.095 | 12 | 7.246 | 0.251 | 0.558 | 0.752 | 1.673 |

| |
|---|
| ^{a} Median CV% was measured from the median value of CVs for each amount point of heavy peptides; ^{b} The concentration of standard EV proteins was measured as 0.0387 mg/ml and applied in calculating LOD, LLOQ concentration. |

### Example 3 Verification of the surrogate peptides in measuring clinical samples

The multiplexed assay setup of Example 2 was applied to quantify the selected targets and CD9 in individual plasma derived EVs collected from 80 healthy control humans and 73 CRC patients (i.e., the test set), and the quantification results were summarized in Table 4. The median values of CV for each target were calculated in triplicate experiments in these 153 samples. Four targets (ALCAM, RHAG, TSPAN33, and TTYH3) exhibited the median CV higher than 20%, whereas the other 9 targets and CD9 showcased reliable precision in the multiplexed assay setup of Example 2 in measuring the plasma derived EVs (data not shown). According to the results summarized in Table 4, six targets (ADAM10, ALCAM, APMAP, CD58, CD59, and TSPAN9) and CD9 could be detectable and quantified in more than 50% (37 cases) of the CRC samples, but two targets (ART4 and SELP) could only be detectable and quantified in less than 25% (18 cases) of the CRC samples. The quantifiable range of these 13 targets and CD9 was from 0.003 ng/ml (for ART4) to 5.50 ng/ml (for CD9) in the HC group, and from 0.13 ng/ml (for ART4) to 7.66 ng/ml (CD9) in the CRC group. The average concentrations of most of the 14 targets in the CRC group were higher than their corresponding LLOQ values, except for ALCAM and SELP (FIG. 1). Based on the results as described above, the present multiplexed assay setup was then applied to quantify the 14 selected targets in plasma derived EVs from both the HC humans and the CRC patients (Table 4). When comparing the levels of the 13 targets between the HC and the CRC groups, 10 targets (ADAM10, ALCAM, APMAP, ART4, CD58, CD59, ICAM3, ITGAM, RHAG, and TSPAN9) were significantly upregulated (1.72-50.49 fold changes; p < 0.05) in the CRC patients. Among them, 3 targets (ADAM10, CD59, and TSPAN9) showed higher ability (values of area under the receiver operating characteristic curve (AUC) = 0.83, 0.95, and 0.87, respectively) in detecting CRC, and were selected for further analysis (Table 4). The results for each of the targets for use in individually measuring the level of the corresponding target proteins in the plasma-EV samples isolated from the HC and the CRC groups were presented in FIGs. 2A-2M.

**Table 4 Concentrations of the indicated proteins in plasma EV samples of HC and CRC**

| **Protein** | **HC (n = 80)** | | **CRC (n = 73)** | | **CRC vs. HC** | | | | |
|---|---|---|---|---|---|---|---|---|---|
| | **ng/ml^{a}** | **Detectable^{b}** | **ng/ml^{a}** | **Detectable^{b}** | **Fold change^{c}** | **p-value^{d}** | **AUC** | **Sensitivity** | **Specificity** |
| ADAM10 | 1.83 ± 3.37 | 40/80 | 4.92 ± 2.41 | 68/73 | 2.69 | < 0.0001 | 0.83 | 93.15 | 77.50 |
| ALCAM | 0.66 ± 1.38 | 31/80 | 1.76 ± 2.58 | 43/73 | 2.65 | 0.0005 | 0.65 | 56.16 | 76.25 |
| APMAP | 0.91 ± 2.91 | 21/80 | 2.64 ± 3.62 | 40/73 | 2.90 | < 0.0001 | 0.67 | 39.73 | 92.50 |
| ART4 | 0.00 ± 0.02 | 2/80 | 0.13 ± 0.33 | 11/73 | 50.49 | 0.0027 | 0.56 | 15.07 | 100.00 |
| CD58 | 0.28 ± 0.72 | 31/80 | 1.54 ± 1.92 | 48/73 | 5.49 | < 0.0001 | 0.72 | 60.27 | 90.00 |
| CD59 | 0.83 ± 0.94 | 77/80 | 4.35 ± 2.93 | 73/73 | 5.26 | < 0.0001 | 0.95 | 100.00 | 82.50 |
| CD9 | 5.50 ± 11.29 | 36/80 | 7.66 ± 7.65 | 46/73 | 1.39 | 0.0035 | 0.63 | 53.42 | 73.75 |
| ICAM3 | 0.93 ± 1.61 | 52/80 | 1.59 ± 2.86 | 22/73 | 1.72 | 0.0424 | 0.59 | 69.86 | 65.00 |
| ITGAM | 0.08 ± 0.22 | 16/80 | 2.72 ± 11.37 | 23/73 | 32.79 | 0.0142 | 0.59 | 31.51 | 98.75 |
| RHAG | 0.10 ± 0.21 | 18/80 | 0.58 ± 2.14 | 33/73 | 5.70 | 0.0059 | 0.61 | 43.84 | 82.50 |
| SELP | 1.19 ± 2.89 | 13/80 | 0.72 ± 1.45 | 15/73 | 0.61 | 0.8257 | 0.51 | 20.55 | 83.75 |
| TSPAN33 | 0.99 ± 1.45 | 37/80 | 4.50 ± 7.81 | 23/73 | 4.53 | 0.9226 | 0.50 | 28.77 | 100.00 |
| TSPAN9 | 1.11 ± 2.06 | 69/80 | 2.42 ± 0.90 | 73/73 | 2.19 | < 0.0001 | 0.87 | 100.00 | 71.25 |
| TTYH3 | 2.52 ± 6.19 | 54/80 | 1.54 ± 2.60 | 31/73 | 0.61 | 0.1323 | 0.57 | 57.53 | 67.50 |
| CEA | 3.03 ± 5.35 | 80/80 | 77.75 ± 469.49 | 73/73 | 25.68 | 0.9906 | 0.50 | 31.51 | 95 |
| ^{a} Mean ± S. D.; ^{b} Detectable (concentration > 0) case number/total case number; ^{c} Fold change of protein levels in the CRC group over the HC group; ^{d} The Mann-Whitney test and statistically significant at p-value < 0.05. | | | | | | | | | |

**Table 5 Concentrations of the indicated proteins in plasma EV samples of HC and CRC at TMN stage I and II**

| **Protein** | **HC (n = 80)** | | **CRC at TNM stage I-II (n = 32)** | | **CRC at TNM stage I-II vs. HC** | | | | |
|---|---|---|---|---|---|---|---|---|---|
| | **ng/ml^{a}** | **Detectable^{b}** | **ng/ml^{a}** | **Detectable^{b}** | **Fold change^{c}** | **p-value^{d}** | **AUC** | **Sensitivity** | **Specificity** |
| ADAM10 | 1.83 ± 3.37 | 40/80 | 4.86 ± 2.29 | 32/32 | 2.66 | < 0.0001 | 0.85 | 100 | 77.5 |
| CD59 | 0.83 ± 0.94 | 77/80 | 3.45 ± 0.78 | 32/32 | 4.16 | < 0.0001 | 0.96 | 100 | 86.25 |
| CD9 | 5.50 ± 11.29 | 36/80 | 7.48 ± 7.44 | 18/32 | 1.36 | 0.037 | 0.62 | 37.5 | 95 |
| TSPAN9 | 1.11 ± 2.06 | 69/80 | 2.14 ± 0.63 | 32/32 | 1.93 | < 0.0001 | 0.86 | 100 | 81.25 |
| CEA | 3.03 ± 5.35 | 80/80 | 1.81 ± 1.81 | 32/32 | 0.6 | 0.0006 | 0.7 | 43.75 | 100 |
| ^{a} Mean ± S.D.; ^{b} Detectable (concentration > 0) case number/total case number; ^{c} Fold change of protein levels in the CRC group over the HC group; ^{d} The Mann-Whitney test and statistically significant at p-value ≦ 0.05. | | | | | | | | | |

Further, the levels of these 3 targets in the early-stage CRC patients (TNM stage I and II, n = 32) and the HC humans were evaluated, and the results were summarized in Table 5. Note that there was significantly increase in the fold changes (CRC/HC) of the three targets (2.66 for ADAM10, 4.16 for CD59, and 1.93 for TSPAN9) and in the AUC values of the three targets (0.85 for ADAM10, 0.96 for CD59, and 0.86 for TSPAN9). These data suggested that all the three targets have highly diagnostic ability for diagnosing early-stage CRC patients (Table 5).

As a supplement, among the 13 selected targets, eight (ADAM 10, ALCAM, CD58, CD59, ITGAM, SELP, TSPAN9, and TTYH3) have been previously reported as potential biomarkers for CRC detection. Nonetheless, according to the results provided above, only three targets (ADAM10, CD59, and TSPAN9) exhibited sufficient power (AUC > 0.8) to discriminate CRC patients from HC humans in the current study (Tables 4-5 and FIGs. 2A-2M), in which a head-to-head quantitative comparison between the 13 selected targets was performed in the same sample set. It is evidenced that without actual experimentation, the power of the above targets to discriminate CRC patients from HC humans cannot be confirmed.

Moreover, it is also noted that the level of CD9 and the EV protein concentration were almost equal among the HC, the CRC, and the early-stage CRC groups, indicating that the fold changes of the 13 targets are not attributable to the amount of EVs (Tables 4-5 and FIGs. 2N-2O). The level of plasma CEA was also examined herein, and the results depicted that only a small fraction of the CRC patients had high level of CEA, while there was no significant difference in the levels of CEA between the HC and the CRC groups (Table 4 and FIG. 2P). Taken together, these data evidenced that ADAM 10, CD59, and TSPAN9 may serve as good biomarkers for plasma EV of CRC.

### Example 4 Generation of the panels of biomarkers for plasma EV protein

### 4.1 Verification of the panels of biomarkers

The purpose of the present example is to establish a panel of biomarker for plasma derived EVs for use in CRC detection, based on the knowledge gained above. To this end, logistic regression analysis was used to process the quantification results acquired above from the five targets (ADAM10, APMAP, CD58, CD59, and TSPAN9) (so as to calculate the predictive probability as a risk score), which could be quantified in more than 50% (37 cases) of the CRC samples (n = 73, in the test set) with their average concentrations higher than their corresponding LLOQ values. Accordingly, the panels of biomarkers containing at least two proteins of ADAM10, CD59, and/or TSPAN9 were generated via the above analysis for detecting CRC, and the ROC curves were generated by the values of the measured probability (FIG. 3A); details for the ROC curves, including the AUC values, the predetermined risk scores, sensitivity, and specificity were summarized in Table 6, and the equations with regards to the indicated panels were provided as follows:

| | |
|---|---|
| (CD59+TSPAN9+ADAM10) | $risk score = \frac{{e}^{\left(-3.815+4.037X1-1.514X2+0.142X3\right)}}{1 + {e}^{\left(-3.815+4.037X1-1.514X2+0.142X3\right)}}$ |
| (CD59+TSPAN9) | $risk score = \frac{{e}^{\left(-3.743+3.780X1-1.596X2\right)}}{1 + {e}^{\left(-3.743+3.780X1-1.596X2\right)}}$ |
| (CD59+ADAM10) | $risk score = \frac{{e}^{\left(-3.620+3.140X1-0.618X2\right)}}{1 + {e}^{\left(-3.620+3.140X1-0.618X2\right)}}$ |
| (TSPAN9+ADAM10) | $risk score = \frac{{e}^{\left(-1.185-0.380X1+0.546X2\right)}}{1 + {e}^{\left(-1.185-0.380X1+0.546X2\right)}}$ |

**Table 6 Details for the ROC curves for discriminating CRC from HC**

| **Proteins** | **AUC** | **Risk score** | **Sensitivity (%)** | **Specificity (%)** |
|---|---|---|---|---|
| CD59+TSPAN9+ADAM10 | 0.98 | 0.238 | 97.26 | 91.25 |
| CD59+TSPAN9 | 0.98 | 0.211 | 98.63 | 91.25 |
| CD59+ADAM10 | 0.97 | 0.458 | 89.04 | 92.50 |
| CD59 | 0.95 | 1.032 | 100.00 | 82.50 |
| TSPAN9 | 0.87 | 0.840 | 100.00 | 71.25 |
| ADAM10 | 0.83 | 2.327 | 93.15 | 77.50 |
| TSPAN9+ADAM10 | 0.81 | 0.387 | 93.15 | 75.00 |
| CEA | 0.50 | 4.6 | 31.51 | 95.00 |

Thus, the ROC analysis for HC vs. CRC samples in the test set indicated that the AUC of the panels of the biomarkers ADAM10, CD59, and/or TSPAN9 were 0.81-0.98 (Table 6). When the cutoff value of the risk score (i.e., the predetermined risk score) was set at the best cutting point for distinguishing HC and CRC groups (i.e., 0.211, 0.238, 0.387, or 0.458, depending on the biomarkers chosen), all the panels of the aforementioned biomarkers gave a high sensitivity (89.04-98.63%) and a high specificity (75-92.5%) in the test set.

Further, the predictivity for discriminating early-stage CRC from HC using the ROC analysis as described above were examined likewise, and the results were as provided in FIG. 3B and Table 7, with the equations with regards to the indicated panels being as follows:

| | |
|---|---|
| (CD59+TSPAN9+ADAM10) | $risk score = \frac{{e}^{\left(-3.815+4.037X1-1.514X2+0.142X3\right)}}{1 + {e}^{\left(-3.815+4.037X1-1.514X2+0.142X3\right)}}$ |
| (CD59+TSPAN9) | $risk score = \frac{{e}^{\left(-3.743+3.780X1-1.596X2\right)}}{1 + {e}^{\left(-3.743+3.780X1-1.596X2\right)}}$ |
| (CD59+ADAM10) | $risk score = \frac{{e}^{\left(-3.620+3.140X1-0.618X2\right)}}{1 + {e}^{\left(-3.620+3.140X1-0.618X2\right)}}$ |
| (TSPAN9+ADAM10) | $risk score = \frac{{e}^{\left(-1.185-0.380X1+0.546X2\right)}}{1 + {e}^{\left(-1.185-0.380X1+0.546X2\right)}}$ |

**Table 7 Details for the ROC curves for discriminating CRC at TMN stage I and II from HC**

| **Proteins** | **AUC** | **Risk score** | **Sensitivity (%)** | **Specificity (%)** |
|---|---|---|---|---|
| CD59+TSPAN9+ADAM10 | 0.99 | 0.238 | 100.00 | 91.25 |
| CD59+TSPAN9 | 0.99 | 0.211 | 100.00 | 91.25 |
| CD59+ADAM10 | 0.98 | 0.458 | 96.88 | 92.50 |
| CD59 | 0.96 | 1.032 | 100.00 | 82.50 |
| TSPAN9 | 0.86 | 0.840 | 100.00 | 71.25 |
| ADAM10 | 0.85 | 2.327 | 100.00 | 77.50 |
| TSPAN9+ADAM10 | 0.86 | 0.387 | 100.00 | 75.00 |

According to the results, the AUC of the panels of the biomarkers ADAM10, CD59, and/or TSPAN9 in discriminating early-stage CRC from HC were 0.86-0.99 (Table 7). Similarly, when the cutoff value of the risk score (i.e., the predetermined risk score) was set at the best cutting point for distinguishing HC and early-stage CRC groups (i.e., 0.211, 0.238, 0.387, or 0.458, depending on the biomarkers chosen), all the panels of the aforementioned biomarkers gave a high sensitivity (96.88-100%) and a high specificity (75-92.5%) in the test set. Taken together, these data demonstrated that the panels of the biomarkers ADAM10, CD59, and/or TSPAN9 exhibited sufficient predictivity in discriminating CRC (or even early-stage CRC) from healthy controls, thus the panels of the above biomarkers may serve as a CRC biomarkers.

### 4.2 Association of the level of the biomarkers in the plasma derived EVs with clinicopathological characteristics of the CRC patients

The potential association of the level of ADAM10, CD59, and /or TSPAN9 in plasma derived EVs, and the plasma CEA, with the following clinicopathological characteristics of the enrolled CRC patients, including gender, age, tumor stage, lymph node metastasis, distant metastasis, and TNM stage, were investigated herein. The results were presented in Table 8, indicating that: (i) the measurements for the four biomarkers (ADAM10, CD59, TSPAN9, and CEA) are not significantly associated with gender or age; (ii) CD59 having higher levels in the plasma-EVs is significantly correlated with distant metastasis (p = 0.0475); (iii) TSPAN9 having higher levels in the plasma-EVs is significantly correlated with lymph node metastasis (p = 0.0011), distant metastasis (p = 0.0104), and higher TNM stage (p = 0.0065); (iv) CEA having higher levels in the plasma is significantly correlated with higher tumor stage (p = 0.0035), lymph node metastasis (p = 0.0003), distant metastasis at diagnosis (p = 0.0252), and higher TNM stage (p = 0.0010); and (v) the level of ADAM10 in the plasma-EVs is not significantly correlated with any of the indicated clinical characteristics (Table 8). These data suggested that although the level of the above biomarkers may or may not correlate with the clinicopathological characteristics of CRC, they still exhibit good detection ability for detecting CRC.

**Table 8 Correlation of the level of ADAM10, CD59, and TSPAN9 in the plasma-EV and the clinicopathological characteristics of the CRC patients**

| **Characteristics** | **Case** No. | **ADAM10 (ng/ml)** | **p-value** | **CD59 (ng/ml)** | **p-value** | **TSPAN9** (ng/ml) | **p-value** | **CEA (ng/ml)** | **p-value** |
|---|---|---|---|---|---|---|---|---|---|
| Gender^{a} | - | - | - | - | - | - | - | - | - |
| Female | 37 | 4.82 ± 2.62 | 0.9781 | 4.38 ± 3.11 | 0.9257 | 2.50 ± 0.91 | 0.3766 | 126.51 ± 657.7 | 0.9715 |
| Male | 36 | 5.02 ± 2.20 | - | 4.32 ± 2.77 | - | 2.33 ± 0.90 | - | 27.63 ± 58.38 | - |
| Age (years)^{a} | - | - | - | - | - | - | - | - | - |
| < 58° | 34 | 4.69 ± 1.81 | 0.5629 | 4.29 ± 2.75 | 0.9494 | 2.38 ± 0.88 | 0.8798 | 10.64 ± 31.55 | 0.2391 |
| ≥ 58 | 39 | 5.12 ± 2.84 | - | 4.41 ± 3.1 | - | 2.45 ± 0.93 | - | 136.24 ± 639.71 | - |
| Tumor stage^{a} | - | - | - | - | - | - | - | - | - |
| T1 | 14 | 4.39 ± 1.36 | 0.4949 | 3.39 ± 0.79 | 0.4428 | 2.07 ± 0.65 | 0.2655 | 0.88 ± 0.53 | 0.0035^{d} |
| T2 | 9 | 5.14 ± 3.63 | - | 3.63 ± 2.72 | - | 2.25 ± 0.84 | - | 23.06 ± 60.24 | - |
| T3 | 42 | 5.15 ± 2.29 | - | 4.81 ± 3.28 | - | 2.62 ± 0.90 | - | 112.46 ± 612.04 | - |
| T4 | 8 | 4.40 ± 3.04 | - | 4.45 ± 3.42 | - | 2.15 ± 1.21 | - | 91.53 ± 214.20 | - |
| Lymph node metastasis^{a} | - | - | - | - | - | - | - | - | - |
| N0 | 37 | 5.02 ± 2.22 | 0.0789 | 3.96 ± 1.83 | 0.1842 | 2.24 ± 0.71 | 0.0011^{d} | 6.16 ± 26.38 | 0.0003^{d} |
| N1 | 36 | 4.82 ± 2.62 | - | 4.76 ± 3.72 | - | 2.60 ± 1.05 | - | 151.32 ± 664.63 | - |
| Distant metastasis^{a} | - | - | - | - | - | - | - | - | - |
| M0 | 57 | 4.84 ± 2.51 | 0.2103 | 4.13 ± 2.84 | 0.0475^{d} | 2.30 ± 0.86 | 0.0104^{d} | 6.97 ± 29.67 | 0.0252^{d} |
| M1 | 16 | 5.18 ± 2.05 | - | 5.15 ± 3.19 | - | 2.85 ± 0.94 | - | 329.87 ± 983.81 | - |
| TNM stage | - | - | - | - | - | - | - | - | - |
| Stage I | 18 | 4.87 ± 2.42 | 0.3214 | 3.50 ± 0.73 | 0.2273 | 2.15 ± 0.64 | 0.0065^{d} | 1.40 ± 1.67 | 0.0010^{d} |
| Stage II | 14 | 4.85 ± 2.20 | - | 3.37 ± 0.86 | - | 2.13 ± 0.63 | - | 2.33 ± 1.91 | - |
| Stage III | 25 | 4.83 ± 2.82 | - | 5.00 ± 4.07 | - | 2.50 ± 1.07 | - | 13.59 ± 44.37 | - |
| Stage IV | 16 | 5.18 ± 2.05 | - | 5.15 ± 3.19 | - | 2.85 ± 0.94 | - | 329.87 ± 983.81 | - |

| | | | | | | | | | |
|---|---|---|---|---|---|---|---|---|---|
| The p-values were generated using ^{a} the Mann-Whitney test (mean ± S.D.) or ^{b} the Kruskal-Wallis test (mean ± S.D.); ^{c} The threshold of age was determined by the median of all patients' age; ^{d} Statistically significant, p-value ≤ 0.05. | | | | | | | | | |

For a successful biomarker verification study, the following criteria such as multiplexed assay, high sensitivity and specificity, and broad dynamic range for detection are required. In the present invention, the protein (peptide) levels in the plasma EVs were analyzed by LC-PIS-MS, which is an established technology for performing both qualitative and quantitative measurements. It enables to detect the 13 candidate protein markers at concentrations ranging from 0.1 ng/ml to 2000 ng/ml. This detection limit is as good as the specificity of an antibody (such as that used in ELISA), but LC-PIS-MS can avoid the bias that could be introduced by off-target antibody effects.

In summary, early detection of CRC is critical for successful and cost-effective disease control and patient management. As there is no suitable molecular marker available for the clinical diagnosis or monitoring of CRC at the moment, the present invention yet by contrast describes the development and validation of a clinically applicable panels of biomarkers in plasma EV proteins for the early detection of CRC and the monitoring of patients with high risk CRC, thereby worthy for patent protection.

## Claims

1. A method for determining whether an human has or is at risk of developing colorectal cancer (CRC) with an extracellular vesicle (EV) derived from an ex vivo biological sample isolated from the human, comprising:
(a) determining a concentration of (1) two target proteins of CD59 and TSPAN9 or (2) three target proteins of ADAM10, CD59, and TSPAN9 in the EV derived from the ex vivo biological sample by the steps of:
(a-1) selecting two or three surrogate peptides corresponding to the two or three target proteins, wherein each of the two or three surrogate peptides is selected from the group consisting of ADAM10 surrogate peptide, CD59 surrogate peptide, and TSPAN9 surrogate peptide, wherein the ADAM10 surrogate peptide comprises the amino acid sequence of SEQ ID NO: 1; the CD59 surrogate peptide comprises the amino acid sequence of SEQ ID NO: 6; and the TSPAN9 surrogate peptide comprises the amino acid sequence of SEQ ID NO: 12;
(a-2) labeling the two or three surrogate peptides of step (a-1) by isotopes;
(a-3) digesting the EV derived from the ex vivo biological sample by means of a proteolytic process to produce a digest;
(a-4) adding a predetermined concentration of the isotope-labeled two or three surrogate peptides of step (a-2) to the digest of step (a-3);
(a-5) determining the amounts of the two or three target peptides and the isotope-labeled two or three surrogate peptides in the mixture of step (a-4) by mass spectrometry;
(a-6) dividing the determined amounts of the two or three target peptides by the determined amounts of the isotope-labeled two or three surrogate peptides to produce a ratio; and
(a-7) determining the concentration of the two or three target proteins in the EV derived from the ex vivo biological sample based on the ratio of step (a-6) and the predetermined concentration of the isotope-labeled two or three surrogate peptides of step (a-4);
(b) calculating a risk score based on the concentrations of the two or three target proteins determined in step (a); and
(c) determining whether the human has or is at risk of developing CRC based on the calculated risk score of step (b), wherein the human does not have or is at low risk of developing CRC if the calculated risk score of step (b) is lower than a predetermined risk score, and the human has or is at high risk of developing CRC if the calculated risk score of step (b) is the same or above the predetermined risk score.

2. The method of claim 1, wherein the risk score is calculated by use of logistic regression.

3. The method of claim 1, wherein the risk score is calculated by an equation of: $risk score = \frac{{e}^{\left(a+b1X1+b2X2+b3X3\right)}}{1 + {e}^{\left(a+b1X1+b2X2+b3X3\right)}}$ wherein e is a mathematical constant that is the base of the natural logarithm; a is a constant value; X1, X2, and X3 respectively represent the concentrations of ADAM 10, CD59, and TSPAN9; and b1, b2, and b3 respectively represent the coefficient of variation of ADAM10, CD59, and TSPAN9.

4. The method of claim 3, wherein
(1) in the case when the two target proteins in step (a) are CD59 and TSPAN9, the predetermined risk score in step (c) is 0.211, or
(2) in the case when the three target proteins in step (a) are ADAM10, CD59, and TSPAN9, and the predetermined risk score in step (c) is 0.238.

5. The method of claim 1, wherein the ex vivo biological sample is selected from the group consisting of blood, plasma, serum, saliva, sputum, urine, ascites, cerebrospinal fluid, amniotic fluid, and tissue lysate.

## Patentansprüche

1. Verfahren zur Feststellung, ob eine Person an Darmkrebs (CRC) erkrankt ist oder ein Risiko hat, daran zu erkranken, mit einem extrazellulären Vesikel (EV), das aus einer ex vivo biologischen Probe stammt, die von der Person isoliert wurde, welches umfasst:
(a) Bestimmen einer Konzentration von (1) zwei Zielproteinen von CD59 und TSPAN9, oder (2) drei Zielproteinen von ADAM10, CD59 und TSPAN9, in den EV, die aus der ex vivo biologischen Probe abgeleitet wurden, durch folgende Schritte:
(a-1) Auswählen von zwei oder drei Ersatzpeptiden, die mit den zwei oder drei Zielproteinen korrespondieren, wobei jedes der zwei oder drei Ersatzpeptide aus der Gruppe ausgewählt ist, die ADAM10-Ersatzpeptid, CD59-Ersatzpeptid und TSPAN9-Ersatzpeptid enthält, wobei das ADAM10-Ersatzpeptid die Aminosäuresequenz von SEQ ID NO: 1 umfasst; das CD59- Ersatzpeptid die Aminosäuresequenz von SEQ ID NO: 6 umfasst; und das TSPAN9- Ersatzpeptid die Aminosäuresequenz von SEQ ID NO: 12 umfasst;
(a-2) Markieren der zwei oder drei Ersatzpeptide aus Schritt (a-1) mit Isotopen;
(a-3) Verdauen des aus der ex vivo biologischen Probe abgeleiteten EV mittels eines proteolytischen Verfahrens, um ein Verdauungsprodukt herzustellen;
(a-4) Zugeben einer vorgegebenen Konzentration der isotopenmarkierten zwei oder drei Ersatzpeptide aus Schritt (a-2) zu dem Verdauungsprodukt aus Schritt (a-3);
(a-5) Bestimmen der Mengen der zwei oder drei Zielpeptide und der isotopenmarkierten zwei oder drei Ersatzpeptide in der Mischung aus Schritt (a-4) durch Massenspektrometrie;
(a-6) Dividieren der ermittelten Mengen der zwei oder drei Zielpeptide durch die ermittelten Mengen der isotopenmarkierten zwei oder drei Ersatzpeptide, um ein Verhältnis zu erhalten; und
(a-7) Bestimmen der Konzentration der zwei oder drei Zielproteine in dem von der ex vivo biologischen Probe abgeleiteten EV basierend auf dem Verhältnis aus Schritt (a-6) und der vorgegebenen Konzentration der isotopenmarkierten zwei oder drei Ersatzpeptide aus Schritt (a-4);
(b) Berechnen eines Risikowerts auf Basis der Konzentrationen der zwei oder drei Zielproteine, die in Schritt (a) bestimmt wurden; und
(c) Bestimmen, ob die Person an Darmkrebs erkrankt ist oder ein Risiko hat, daran zu erkranken, basierend auf dem berechneten Risikowert aus Schritt (b), wobei die Person keinen Darmkrebs hat oder ein geringes Risiko für die Entwicklung desselben aufweist, falls der berechnete Risikowert aus Schritt (b) kleiner als ein vorgegebener Risikowert ist, und die Person Darmkrebs hat oder ein hohes Risiko für die Entwicklung desselben aufweist, falls der berechnete Risikowert aus Schritt (b) gleich dem oder höher als der vorgegebene Risikowert ist.

2. Verfahren nach Anspruch 1, bei welchem der Risikowert unter Verwendung einer logistischen Regression berechnet wird.

3. Verfahren nach Anspruch 1, bei welchem der Risikowert nach folgender Gleichung berechnet wird: $Risikowert = \frac{{e}^{\left(a+b1X1+b2X2+b3X3\right)}}{1 + {e}^{\left(a+b1X1+b2X2+b3X3\right)}}$ wobei e eine mathematische Konstante ist, die die Basis des natürlichen Logarithmus ist; a ein konstanter Wert ist; X1, X2 und X3 jeweils die Konzentrationen von ADAM10, CD59 und TSPAN9 repräsentieren; und b1, b2 und b3 jeweils den Variationskoeffizienten von ADAM10, CD59 und TSPAN9 repräsentieren.

4. Verfahren nach Anspruch 3, bei welchem
(1) in dem Fall, wenn die zwei Zielproteine in Schritt (a) CD59 und TSPAN9 sind, der vorgegebene Risikowert in Schritt (c) 0,211 ist, oder
(2) in dem Fall, wenn die drei Zielproteine in Schritt (a) ADAM10, CD59 und TSPAN9 sind, der vorgegebene Risikowert in Schritt (c) 0,238 ist.

5. Verfahren nach Anspruch 1, bei welchem die ex vivo biologische Probe aus einer Gruppe ausgewählt ist, die Blut, Plasma, Serum, Speichel, Sputum, Urin, Aszites, Rückenmarksflüssigkeit, Fruchtwasser und Gewebelysat enthält.

## Revendications

1. Procédé pour déterminer si un humain présente ou s'expose à un risque de développer un cancer colorectal (CRC) avec une vésicule extracellulaire (EV) dérivée d'un échantillon biologique ex vivo isolé de l'humain, comprenant :
(a) la détermination d'une concentration (1) de deux protéines cibles de CD59 et TSPAN9 ou (2) de trois protéines cibles d'ADAM10, CD59 et TSPAN9 dans l'EV dérivée de l'échantillon biologique ex vivo par les étapes de :
(a-1) sélection de deux ou trois peptides de substitution correspondant aux deux ou trois protéines cibles, dans lequel chacun des deux ou trois peptides de substitution est sélectionné à partir du groupe constitué par le peptide de substitution ADAM10, le peptide de substitution CD59 et le peptide de substitution TSPAN9, dans lequel le peptide de substitution ADAM10 comprend la séquence d'acides aminés SEQ ID NO: 1; le peptide de substitution CD59 comprend la séquence d'acides aminés SEQ ID NO: 6 ; et le peptide de substitution TSPAN9 comprend la séquence d'acides aminés SEQ ID NO: 12 ;
(a-2) étiquetage des deux ou trois peptides de substitution de l'étape (a-1) par des isotopes ;
(a-3) direction de l'EV dérivée de l'échantillon biologique ex vivo au moyen d'un processus protéolytique pour produire un digest ;
(a-4) ajout d'une concentration prédéterminée des deux ou trois peptides de substitution marqués par isotope de l'étape (a-2) au digest de l'étape (a-3) ;
(a-5) détermination des quantités des deux ou trois peptides cibles et des deux ou trois peptides de substitution marqués par isotope dans le mélange de l'étape (a-4) par spectrométrie de masse ;
(a-6) division des quantités déterminées des deux ou trois peptides cibles par les quantités déterminées des deux ou trois peptides de substitution marqués par isotope pour produire un rapport ; et
(a-7) détermination de la concentration des deux ou trois protéines cibles dans l'EV dérivée de l'échantillon biologique ex vivo sur la base du rapport de l'étape (a-6) et de la concentration prédéterminée des deux ou trois peptides de substitution marqués par isotope de l'étape (a-4) ;
b) calcul d'un score de risque sur la base des concentrations des deux ou trois protéines cibles déterminées à l'étape (a) ; et
(c) détermination si l'être humain présente ou s'expose à un risque de développer un CRC sur la base du score de risque calculé à l'étape (b), dans lequel l'humain n'a pas ou présente un faible risque de développer un CRC si le score de risque calculé à l'étape (b) est inférieur à un score de risque prédéterminé, et l'humain présente ou s'expose à un risque élevé de développer un CCR si le score de risque calculé à l'étape (b) est le même ou supérieur au score de risque prédéterminé.

2. Procédé selon la revendication 1, dans lequel le score de risque est calculé en utilisant une régression logistique.

3. Procédé selon la revendication 1, dans lequel le score de risque est calculé par une équation de : $score de risque = \frac{{e}^{\left(a+b1X1+b2X2+b3X3\right)}}{1 + {e}^{\left(a+b1X1+b2X2+b3X3\right)}}$ dans lequel e est une constante mathématique qui est la base du logarithme naturel ; a est une valeur constante ; X1, X2 et X3 représentent respectivement les concentrations d'ADAM10, de CD59 et de TSPAN9 ; et b1, b2 et b3 représentent respectivement le coefficient de variation d'ADAM10, de CD59 et de TSPAN9.

4. Procédé selon la revendication 3, dans lequel
(1) dans le cas où les deux protéines cibles de l'étape (a) sont CD59 et TSPAN9, le score de risque prédéterminé de l'étape (c) est de 0,211, ou
(2) dans le cas où les trois protéines cibles de l'étape (a) sont ADAM10, CD59 et TSPAN9, et le score de risque prédéterminé de l'étape (c) est de 0,238.

5. Procédé selon la revendication 1, dans lequel l'échantillon biologique ex vivo est sélectionné parmi le groupe constitué de sang, de plasma, de sérum, de salive, d'expectorations, d'urine, d'ascite, de liquide céphalo-rachidien, de liquide amniotique et de lysat tissulaire.
